# EUROPEAN PATENT SPECIFICATION

(11) **EP 3 535 392 B1**
(45) Date of publication and mention of the grant of the patent: **27.03.2024**
(21) Application number: 17797585.1
(22) Date of filing: 30.10.2017
(51) Int. Cl.: A61K 35/00

(54) **IMMUNOLOGICALLY DISCERNIBLE CELL SURFACE VARIANTS FOR USE IN CELL THERAPY**
IMMUNOLOGISCH ERKENNBARE ZELLOBERFLÄCHENVARIANTEN ZUR VERWENDUNG IN DER ZELLTHERAPIE
VARIANTS DE SURFACE CELLULAIRE DISCERNABLES SUR LE PLAN IMMUNOLOGIQUE DESTINÉS À ÊTRE UTILISÉS EN THÉRAPIE CELLULAIRE

(30) Priority: 02.11.2016 EP 16196860; 02.11.2016 EP 16196858; 25.04.2017 WO PCT/EP2017/059799; 23.10.2017 EP 17197820
(43) Date of publication of application: 11.09.2019
(60) Divisional application: 24155910.3
(73) Proprietor: Universität Basel, 4001 Basel (CH)
(72) Inventor: JEKER, Lukas, 4125 Riehen (CH); KORNETE, Mara, 4055 Basel (CH); BORDOLI SCHWEDE, Lorenza, 4102 Binningen (CH); SCHWEDE, Torsten, 4102 Binningen (CH); LEPORE, Rosalba, 4051 Basel (CH); MATTER MARONE, Romina, 4125 Riehen (CH); RECHER, Mike, 4052 Basel (CH)
(74) Representative: Plasseraud IP
(86) International application number: PCT/EP2017/077826
(87) International publication number: WO 2018/083071

(56) References cited:
- WO-A1-2014/201561
- WO-A1-2017/079400
- WO-A1-2017/186718
- US-A1- 2006 193 857
- US-A1- 2011 021 759
- US-A1- 2016 144 026
- KAWAKAMI Y ET AL: "SOMATIC GENE THERAPY FOR BIABETES WITH AN IMMUNOLOGICAL SAFETY SYSTEM FOR COMPLETE REMOVAL OF TRANSPLANTED CELLS", DIABETES, AMERICAN DIABETES ASSOCIATION, US, vol. 41, no. 8, 1 August 1992 (1992-08-01) , pages 956-961, XP000196802, ISSN: 0012-1797, DOI: 10.2337/DIABETES.41.8.956
- SUSHMITA ROY ET AL: "Donor Hematopoietic Stem Cells Confer Long-Term Marrow Reconstitution by Self-Renewal Divisions Exceeding to That of Host Cells", PLOS ONE, vol. 7, no. 12, 5 December 2012 (2012-12-05), page e50693, XP055436532, DOI: 10.1371/journal.pone.0050693
- GREGORY M. FINDLAY ET AL: "Saturation editing of genomic regions by multiplex homology-directed repair", NATURE, vol. 513, no. 7516, 3 September 2014 (2014-09-03), pages 120-123, XP055196389, ISSN: 0028-0836, DOI: 10.1038/nature13695
- K. YOSHIMI ET AL: "Allele-specific genome editing and correction of disease-associated phenotypes in rats using the CRISPR-Cas platform", NATURE COMMUNICATIONS, vol. 5, 26 June 2014 (2014-06-26), XP055196554, DOI: 10.1038/ncomms5240 -& K. YOSHIMI ET AL: "Supplementary information: Allele-specific genome editing and correction of disease-associated phenotypes in rats using the CRISPR-Cas platform", NATURE COMMUNICATIONS, vol. 5, 26 June 2014 (2014-06-26), pages 1-17, XP055243924, DOI: 10.1038/ncomms5240

## Description

The present disclosure relates to the use of cells having a mutant but functional cell surface protein in medical applications wherein selective depletion or enrichment of cell populations is desirable. Mutant but functional cell surface proteins may be introduced into cells by gene editing methods including homology directed repair of DNA double strand breaks, in particular using CRISPR/Cas gene editing, or by the use of base editors. The disclosure further relates to an agent and a method for selective depletion of edited cells in vivo.

Cellular therapy is very powerful therapy option, but often associated with severe unwanted side effects. Transgenes and/or genetic engineering of the transferred cells can cause malignant transformation. Transfer of CAR-T cells can lead to severe on- and off-target effects (cytokine release syndrome) and transfer of allogeneic T cells can cause graft versus host disease (GvHD). The success of cellular therapy in oncology will likely boost cell therapies for other indications, including non-malignant diseases. To increase the safety of cellular therapies, particularly if used in the treatment of non-lethal diseases, it is important to ensure that transferred cells remain safe for years after transfer. In instances where severe unwanted side effects develop, a possibility to selectively deplete the transferred cells via a "safety or kill switch" would significantly increase the safety of cellular therapy.

US2016/144026A1 refers to combination immunotherapy of antigen-recognizing receptors and hematopoietic cells for the treatment of diseases.

The problem underlying the present disclosure is to provide a system that serves to permanently mark and track cells and allows the selective depletion of the marked or unmarked cells in vitro or in vivo. These problems are solved by the subject-matter of the independent claims.

### Description

The Invention is defined in the claims.

A first aspect of the disclosure relates, but not as part of the claimed invention, to a mammalian cell, expressing a first isoform of a surface protein, wherein said first isoform of said surface protein is functionally indistinguishable, but immunologically distinguishable from a second isoform of said surface protein, for use in a medical treatment of a patient having cells expressing said second form of said surface protein.

The expression "immunologically distinguishable" refers to a first and a second isoform of the surface protein that can be distinguished by a ligand specifically binding to either the first or the second isoform.

In the context of the present specification, the expression "specific binding" refers to binding with a dissociation constant K_{D} ≤ 10 E -7.

In the context of the present specification, the expression "ligand" relates to an antibody or an antibody-like molecule. The antibody or antibody-like molecule may be coupled to another molecule (e.g. in an immuno toxin) or may be present on the surface of a cell, in particular an immune cell.

In the context of the present specification, the term "antibody" is used in its meaning known in the art of cell biology and immunology; it refers to whole antibodies including but not limited to immunoglobulin type G (IgG), type A (IgA), type D (IgD), type E (IgE) or type M (IgM), any antigen binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region (VH) and a heavy chain constant region (CH). The heavy chain constant region is comprised of three domains, CH1, CH2 and CH3. Each light chain is comprised of a light chain variable region (abbreviated herein as VL) and a light chain constant region (CL). The light chain constant region is comprised of one domain, CL. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system.

The term "antibody-like molecule" in the context of the present specification refers to a molecule capable of specific binding to another molecule or target with high affinity / a K_{D} ≤ 10E-7 mol/l, in particular a K_{D} ≤ 10E-8 mol/l. An antibody-like molecule binds to its target similarly to the specific binding of an antibody. The term antibody-like molecule encompasses a repeat protein, such as a designed ankyrin repeat protein (Molecular Partners, Zürich), a polypeptide derived from armadillo repeat proteins, a polypeptide derived from leucine-rich repeat proteins, an affimer, an antibody-derived molecule, such as a chimeric antigen receptor (CAR) and a polypeptide derived from tetratricopeptide repeat proteins.

The term antibody-like molecule further encompasses a polypeptide derived from protein A domains, a polypeptide derived from fibronectin domain FN3, a polypeptide derived from consensus fibronectin domains, a polypeptide derived from lipocalins, a polypeptide derived from Zinc fingers, a polypeptide derived from Src homology domain 2 (SH2), a polypeptide derived from Src homology domain 3 (SH3), a polypeptide derived from PDZ domains, a polypeptide derived from gamma-crystallin, a polypeptide derived from ubiquitin, a polypeptide derived from a cysteine knot polypeptide and a polypeptide derived from a knottin.

Ideally, the first and the second isoform of the surface protein can be distinguished by two ligands, wherein one ligand is able to specifically recognize the first isoform and the other ligand is able to specifically recognize the second isoform. In other words, each ligand is able to specifically bind to one isoform, but not to the other isoform. In other words, the ligands are able to discriminate between the two isoforms by specifically binding only one isoform, but not the other one.

The expression "functionally indistinguishable" refers to a first and a second isoform that are equally capable of performing the same function within a cell without significant impairment. In other words, the first and the second isoform are functionally largely indistinguishable. In certain embodiments, a slight functional impairment can be acceptable.

In the context of the present specification, the expressions "first and/or second isoform of the cell surface protein" refer to a first and a second allele of the cell surface protein.

In certain embodiments, the mammalian cell is a human cell.

In certain embodiments, the second isoform of the surface protein relates to the wildtype form of the protein (in other words: the form that usually occurs in nature) and the first isoform relates to an isoform obtained by introducing a mutation in the nucleic acid sequence encoding the second isoform.

In certain embodiments, the second isoform of the surface protein is the native isoform and the first isoform is a genetically engineered isoform derived from the native isoform.

In the context of the present specification, the expression "native protein" refers to a protein that is encoded by a nucleic acid sequence within the genome of the cell, wherein this nucleic acid sequence has not been inserted or mutated by genetic manipulation. In other words, a native protein is a protein that is not a transgenic protein or a genetically engineered protein.

In certain embodiments, the surface protein comprises an extracellular polypeptide sequence and said first isoform comprises an insertion, deletion and/or substitution of 1, 2, 3, 4, or 5, amino acids in comparison to said second isoform.

In certain embodiments, the surface protein comprises an extracellular polypeptide sequence and said first isoform comprises an insertion, deletion and/or substitution of 1-5 amino acids in comparison to said second isoform.

In certain embodiments, the surface protein comprises an extracellular polypeptide sequence and said first isoform comprises an insertion, deletion and/or substitution of 1 amino acid in comparison to said second isoform.

In certain embodiments, the insertion, deletion and/or substitution is positioned at a site that is non-conserved between different mammalian species.

In certain embodiments, the insertion, deletion and/or substitution does not result in a secondary structure change in the surface protein.

In certain embodiments, the insertion, deletion and/or substitution is located at a site that is accessible to ligand binding according to crystal structure analysis or computer-aided structure prediction.

In certain embodiments, the insertion, deletion and/or substitution is located at a site that has a unique topology compared to other mammalian proteins according to crystal structure analysis or computer-aided structure prediction.

Within the context of the present specification, the expression "unique topology" refers to a topology that is only present in the surface protein to be modified by an insertion, deletion and/or substitution of 1-20 amino acids and not in other mammalian proteins, in particular not in other human surface proteins. The presence of the same or a very similar topology in other proteins would hinder the generation of a specific antibody recognizing an epitope located at said site.

In certain embodiments, the insertion, deletion and/or substitution is not located at a site involved in a predicted or experimentally established or confirmed protein-protein interaction of the surface protein.

In certain embodiments, the insertion, deletion and/or substitution does not result in deleting or introducing a disulphide bond inter- or intramolecular interaction, or a hydrophobic stacking. In instances where the insertion, deletion and/or substitution results in deleting a salt bridge inter- or intramolecular interaction, it has to be confirmed that this deletion of a salt bridge interaction is compensated by new interactions within the protein. Otherwise, the deletion of a salt bridge interaction should be avoided.

In certain embodiments, the insertion, deletion and/or substitution does not result in deleting or introducing a posttranslational protein modification site, particularly a glycosylation site, which is important for protein folding.

In certain embodiments, the insertion, deletion and/or substitution does result in deleting or introducing a posttranslational protein modification site, particularly a glycosylation site, which is not relevant for protein folding, thereby creating a new epitope.

In certain embodiments, the first isoform can be distinguished from the second isoform by antibody binding.

In certain embodiments of the present disclosure, the first isoform can be distinguished from the second isoform by reaction of an immune effector cell bearing an antibody. In certain embodiments of the present disclosure, the first isoform can be distinguished from the second isoform by reaction of an immune effector cell bearing an antibody-like molecule. In certain embodiments of the present disclosure, the first isoform can be distinguished from the second isoform by reaction of an immune effector cell bearing an antibody. In certain embodiments of the present disclosure, the first isoform can be distinguished from the second isoform by reaction of an immune effector cell bearing an antibody-like molecule. In certain embodiments of the present disclosure, the first isoform can be distinguished from the second isoform by reaction of a T cell, in particular an activated T cell, bearing a chimeric antigen receptor (CAR). In the context of the present specification, a chimeric antigen receptor (CAR) relates to an engineered receptor which grafts a binding specificity, in particular the specificity of a monoclonal antibody, onto a T cell. A common form of a CAR comprises an extracellular domain derived from a monoclonal antibody having the desired binding specificity, a transmembrane domain and in intracellular domain (Gill and June, Imm Rev, 2014). The extracellular domain comprises a single chain variable fragment comprising the variable regions of an immunoglobulin heavy and light chain. The nucleotide sequence encoding the extracellular domain of such a CAR can be derived from hybridoma cells producing the antibody with the desired binding specificity (Gill and June, Imm Rev, 2014; Fields, Nat Prot, 2013).

In certain embodiments, the surface protein is selected from CD1a, CD1b, CD1c, CD1e, CD1e, CD2, CD3, CD3d, CD3e, CD3g, CD4, CD5, CD6, CD7, CD8a, CD8b, CD9, CD10, CD11a, CD11b, CD11c, CD11d, CDwI2, CD13, CD14, CD15, CD15u, CD15s, CD15su, CD16, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD45, CD45RA, CD45RB, CD45RC, CD45RO, CD46, CD47, CD48, CD49a, CD49b, CD49c, CD49d, CD49e, CD49f, CD50, CD51, CD52, CD53, CD54, CD55, CD56, CD57, CD58, CD59, CD60a, CD60b, CD60c, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CD75, CD75s, CD77, CD79a, CD79b, CD80, CD81, CD82, CD83, CD84, CD85a, CD85d, CD85j, CD85k, CD86, CD87, CD88, CD89, CD90, CD91, CD92, CD93, CD94, CD95, CD96, CD97, CD98, CD99, CD99R, CD100, CD101, CD102, CD103, CD104, CD105, CD106, CD107a, CD107b, CD108, CD109, CD110, CD111, CD112, CD113, CD114, CD115, CD116, CD117, CD118, CD119, CD120a, CD120b, CD121a, CD121b, CD122, CD123, CD124, CD125, CD126, CD127, CD129, CD130, CD131, CD132, CD133, CD134, CD135, CD136, CD137, CD138, CD139, CD140a, CD140b, CD141, CD142, CD143, CD144, CDw145, CD146, CD147, CD148, CDw149, CD150, CD151, CD152, CD153, CD154, CD155, CD156a, CD156b, CD156c, CD157, CD158e, CD158i, CD158k, CD159a, CD159c, CD160, CD161, CD162, CD163, CD164, CD165, CD166, CD167a, CD167b, CD168, CD169, CD170, CD171, CD172a, CD172b, CD172g, CD173, CD174, CD175, CD175s, CD176, CD177, CD178, CD179a, CD179b, CD180, CD181, CD182, CD183, CD184, CD185, CD186, CD191, CD192, CD193, CD194, CD195, CD196, CD197, CDw198, CD199, CD200, CD201, CD202b, CD203c, CD204, CD205, CD206, CD207, CD208, CD209, CD210, CDw210b, CD212, CD213a1, CD213a2, CD215, CD217a, CD218a, CD218b, CD220, CD221, CD222, CD223, CD224, CD225, CD226, CD227, CD228, CD229, CD230, CD231, CD232, CD233, CD234, CD235a, CD235b, CD236, CD236R, CD238, CD239, CD240CE, CD240DCE, CD240D, CD241, CD242, CD243, CD244, CD245, CD246, CD247, CD248, CD249, CD252, CD253, CD254, CD256, CD266, CD267, CD268, CD269 (BCMA), CD270, CD271, CD272, CD273, CD274, CD275, CD276, CD277, CD278, CD279, CD280, CD281, CD282, CD283, CD284, CD286, CD289, CD290, CD292, CDw293, CD294, CD295, CD296, CD297, CD298, CD299, CD300a, CD300c, CD300e, CD301, CD302, CD303, CD304, CD305, CD306, CD307a, CD307b, CD307c, CD307d, CD307e, CD308, CD309, CD312, CD314, CD315, CD316, CD317, CD318, CD319, CD320, CD321, CD322, CD324, CD325, CD326, CD327, CD328, CD329, CD331, CD332, CD333, CD334, CD335, CD336, CD337, CD338, CD339, CD340, CD344, CD349, CD350, CD351, CD352, CD353, CD354, CD355, CD357, CD358, CD360, CD361, CD362, CD363, CD364, CD365, CD366, CD367, CD368, CD369, CD370, CD371, an immunoglobulin light chain (lambda or kappa), a HLA protein and β2-microglobulin.

In the context of the present specification, HLA refers to "human leukocyte antigen" and includes HLA-A, HLA.B, HLA-C, HLA-E, HLA-F, HLA-G, HLA-DM, HLA-DO, HLA-DP, HLA-DQ and HLA-DR.

In certain embodiments, the surface protein is selected from CD2, CD3, CD4, CD5, CD8, CD19, CD20, CD22, CD23, CD33, CD34, CD90, CD45, CD123, CD269 (BCMA), an immunoglobulin light chain (lambda or kappa), a HLA protein and β2-microglobulin.

In certain embodiments, the surface protein is selected from CD45, CD3, CD4, CD8a, CD8b and CD279.

In certain embodiments, the surface protein is selected from CD45, CD45RA and CD45RO.

In certain embodiments, the surface protein is selected from CD45, CD34, CD38, CD59, CD90 and CD117.

In certain embodiments, the surface protein is selected from CD45, CD19, CD20, CD22, CD22, CD23, CD38, CD138, CD268, CD269 (BCMA) and CD319.

In certain embodiments, the surface protein is selected from CD5, CD19, CD20, CD33, CD123, CD38 and CD269.

In certain embodiments, the surface protein is selected from CD45, CD19, CD4 and CD8.

In certain embodiments, the surface protein is CD45.

In certain embodiments, the surface protein is Thy1 (CD90).

In certain embodiments, the surface protein is CD19.

Within the context of the present specification, "Thy1" refers to "thymus cell antigen 1", theta; alternative name: CD90; UniProt ID P04216 (human).

Within the context of the present specification, "CD45" refers to "protein tyrosine phosphatase, receptor type, C (Ptprc)"; UniProt ID P08575 (human).

In the animal experiments of the examples section, CD45 and CD90 refer to the murine homologs of the human genes and proteins, respectively.

In certain embodiments, the surface protein is CD4. In certain embodiments, the surface protein is CD2. In certain embodiments, the surface protein is CD8. In certain embodiments, the surface protein is a HLA protein.

In certain embodiments, the first isoform of the surface protein is not encoded in the patient's native genomic DNA.

In certain embodiments, the cell is an allogeneic cell. An allogeneic cell refers to a cell derived from a donor that is genetically similar to the person receiving the cell. The donor may be a related or unrelated person.

In certain embodiments, the cell is an autologous cell. An allogeneic cell refers to a cell derived from the same person that is receiving the cell.

In certain embodiments, the first isoform was obtained by changing a sequence encoding said surface protein in the patient's native genomic DNA, resulting in inducing said insertion, deletion and/or substitution of amino acids.

In certain embodiments of the present disclosure, the first isoform was obtained by changing the mRNA encoding said surface protein by RNA editing techniques (Zhang, 2017). This method leaves the genomic DNA unchanged, but results in an insertion, deletion and/or substitution of amino acids in the amino acid sequence of the surface protein.

In certain embodiments, the first isoform was obtained by inducing an insertion, deletion and/or substitution of 1, 2, 3, 4 or 5 amino acids in the amino acid sequence of said second isoform of said surface protein.

In certain embodiments, the first isoform was obtained by inducing an insertion, deletion and/or substitution of 1-5 amino acids in the amino acid sequence of said second isoform of said surface protein.

In certain embodiments, the first isoform was obtained by inducing an insertion, deletion and/or substitution of 1 amino acid in the amino acid sequence of said second isoform of said surface protein.

The insertion, deletion and/or substitution of amino acids can be effected by changing the sequence encoding said surface protein in the patient's native genomic DNA (gene editing). Both forms of editing result in a change in the amino acid sequence of the protein.

In certain embodiments, the insertion, deletion and/or substitution is effected by
a. comparing (aligning) one or several homologous sequences of different mammalian species, particularly mouse, rat, primate and human homologues, and positioning the insertion, deletion and/or substitution in a non-conserved site;
b. choosing the insertion, deletion and/or substitution so that crystal structure analysis or computer-aided structure prediction does not predict a secondary structure change of the tract subjected to the insertion, deletion and/or substitution;
c. choosing the insertion, deletion and/or substitution so that it is located at a site that is accessible to ligand binding according to crystal structure analysis or computer-aided structure prediction;
d. choosing the insertion, deletion and/or substitution in a sequence not involved in a predicted or experimentally established protein-protein interaction of the surface protein;
e. choosing the insertion, deletion and/or substitution without deleting or introducing disulphide bond inter- or intramolecular interaction, or hydrophobic stacking; or
f. choosing the insertion, deletion and/or substitution without deleting or introducing a posttranslational protein modification site, particularly a glycosylation site, important for protein folding.

In certain embodiments, the insertion, deletion and/or substitution is effected by
a. comparing (aligning) one or several homologous sequences of different mammalian species, particularly mouse, rat, primate and human homologues, and positioning the insertion, deletion and/or substitution in a non-conserved site;
b. choosing the insertion, deletion and/or substitution so that crystal structure analysis or computer-aided structure prediction does not predict a secondary structure change of the tract subjected to the insertion, deletion and/or substitution;
c. choosing the insertion, deletion and/or substitution so that it is located at a site that is accessible to ligand binding according to crystal structure analysis or computer-aided structure prediction;
d. choosing the insertion, deletion and/or substitution in a sequence not involved in a predicted or experimentally established protein-protein interaction of the surface protein;
e. choosing the insertion, deletion and/or substitution without deleting or introducing disulphide bond inter- or intramolecular interaction, or hydrophobic stacking; and
f. choosing the insertion, deletion and/or substitution without deleting or introducing a posttranslational protein modification site, particularly a glycosylation site, important for protein folding.

In certain embodiments, the insertion, deletion and/or substitution is chosen so that it is located at a site that has a unique topology compared to other mammalian proteins according to crystal structure analysis or computer-aided structure prediction.

A non-conserved site in the context of the present specification relates to a site that is frequently subject to mutations over evolutionary time, as estimated from multiple sequence alignments (MSA) of large numbers of homologous sequences. Site-specific conservation is indicative of the presence of functional or structural constraints acting at specific sites, allowing evaluation of their importance in preserving the structure or the function of a protein. Solvent accessibility state at each site, predicted or observed from available experimentally determined structures, can be used to distinguish between structurally important sites, which are often highly conserved and buried, from functionally important sites, involved in ligand binding, substrate binding, or protein-protein interactions, which are highly conserved and exposed. Hence, a non-conserved site in the context of the present specification relates to a site showing low evolutionary conservation and high solvent accessibility.

Methods for searching similar sequences are well established and routinely used to infer homology, and these include the widely used BLAST tool as well as more sensitive profile-based or Hidden Markov Models based methods such as PSI-BLAST, HMMER, HHblits. Multiple sequence alignments of homologous sequences can be obtained by efficient combination of local and global alignment information as implemented in T-COFFEE or, when possible, by incorporating structural information to guide MSA construction (MAFFT, PROMALS3D, 3D Coffee).

Methods for efficient estimation of site-specific conservation from a MSA are based on Shannon entropy measures, similarity based matrices (i.e. BLOSUM), or probabilistic evolutionary models like the Maximum likelihood and empirical Bayes paradigms (Rate4Site).

Efficient methods for three-dimensional protein structure prediction include but are not limited to comparative based methods, such as SWISS-MODEL, MODELLER, RaptorX and IntFOLD.

Methods for predicting B-cell epitopes use amino acid physicochemical properties, i.e. hydrophobicity, flexibility, polarity, and exposed surface to provide a residue-based probability to be part of either a discontinuous or linear epitope. These tools include but are not limited to: Ellipro, SEPPA, BepiPred, ABCpred, DiscoTope, EpiSearch.

In certain embodiments, the insertion, deletion and/or substitution is located in the extracellular portion of said first surface protein, particularly in an extracellular loop. Mutations in extracellular loops are less likely to affect protein function.

In instances where an antibody or antibody-like molecule reactive against a surface protein of interest already exists, the information which epitope of the surface protein of interest is recognized by this antibody can be used to select the site of the insertion, deletion and/or substitution. The isoform of the protein of interest comprising the epitope to which the existing antibody binds would then correspond to the second isoform, and the newly engineered isoform of the protein of interest comprising the engineered epitope would then correspond to the second isoform.

CDR modelling is described in Messih, Bioinformatics, 2014.

In certain embodiments, the cell is administered prior to, concomitant with or after specific ablation of cells expressing said second isoform of said surface protein. In certain embodiments, the ablation of cells expressing said second isoform of said surface protein is performed by administration to said patient of an antibody.

In certain embodiments, the cell expresses an antibody or an antibody-like molecule reactive against the second isoform of said surface protein.

In certain embodiments, the cell expresses a chimeric antigen receptor reactive against the second isoform of said surface protein.

In certain embodiments, the cell expresses an antibody or an antibody-like molecule, in particular a chimeric antigen receptor, reactive against the second isoform of the surface protein, and the surface protein is selected from CD45, CD19, CD8 and CD4, in particular the surface protein is CD45.

In certain embodiments, the cell expresses
a. a first isoform of a first surface protein, wherein said first isoform of said first surface protein is functionally indistinguishable, but immunologically distinguishable from a second isoform of said first surface protein, and
b. a first isoform of a second surface protein, wherein said first isoform of said second surface protein is functionally indistinguishable, but immunologically distinguishable from a second isoform of said second surface protein.

In certain embodiments, the first surface protein is CD19 and the second surface protein is CD45.

In certain embodiments, the mammalian cell is selected from the group comprising a hematopoietic stem cell (hemocytoblast), a CD4+ T cell, a CD8+ T cell, a memory T cell, a regulatory T cell (T reg), a natural killer cell (NK), an innate lymphoid cell (ILC), a dendritic cell (DC), a B-lymphocyte, a mucosal-associated invariant T cell (MAIT) and a gamma delta T cell (γδ T).

In certain embodiments, the mammalian cell is a hematopoietic cell. In certain embodiments, the mammalian cell is a hematopoietic stem cell. In certain embodiments, the mammalian cell is an immune cell. In certain embodiments, the mammalian cell is a T cell. In certain embodiments, the mammalian cell is a B cell.

In certain embodiments, the cell comprises a genetic alteration correcting or counteracting a disease-related gene defect present in said patient. The expression "genetic alteration correcting a disease-related gene defect" is meant to encompass the insertion of a transgene, a genetic correction of a disease-related mutation, the deletion of a gene, in particular of a gene carrying a disease-related mutation, a change in an epigenetic modification important for the expression of a gene, or a combination thereof. The expression "deletion of a gene" is meant to encompass functional deletion of a gene, in other words preventing the expression of a gene, e.g. by inserting a premature stop codon or by inserting a regulatory repressor sequence.

In certain embodiments, the cell comprises a transgene. In certain embodiments, the transgene is a nucleic acid sequence encoding a functional isoform of the protein affected by the disease-related gene defect. The transgene does not carry the disease-related gene defect.

In certain embodiments, the cell comprises a genetic correction of a disease-related mutation. In instances where the cell comprises a genetic correction of a disease-related gene defect, a disease-causing mutation in a native gene was corrected using gene editing techniques.

In the context of the present specification, gene editing or genetic engineering relates to techniques effecting the insertion, deletion or replacement of a nucleic acid sequence in the genome of a living organism. Gene editing involves site-specific double strand breaks or site specific single strand breaks (nicks) in the genomic DNA. By way of non-limiting example, gene editing can be effected by HDR following CRISPR/Cas-mediated site-specific double strand breaks or by the use of site-specific base editors.

In certain embodiments, the disease-related gene defect is a mutation in a gene selected from the *Foxp3* gene, the CD25 gene, the *Stat5b* gene, the *Stat1* gene and the *Itch* gene.

In certain embodiments, the disease-related gene defect is a mutation in the *Foxp3* gene.

Certain embodiments relate to instances where a graft-versus-host disease (GvHD) is developed following administration of the mammalian cell to the patient. In these instances, the cell is specifically ablated by administration to the patient of an antibody specifically binding to said first isoform (but not to said second isoform) of said cell surface protein.

In certain embodiments, the treatment comprises hematopoietic stem cell transplantation.

In certain embodiments, the treatment comprises transplantation of T cells (in other words: T cell therapy).

In certain embodiments, the treatment comprises organ transplantation.

In certain embodiments, the treatment relates to the treatment of a genetic hematopoietic disease. A non-limiting example of genetic hematopoietic diseases are thalassemias.

In certain embodiments, the treatment relates to the treatment of a T cell mediated disease, in particular a genetic T cell mediated disease, more particularly an IPEX-like syndrome, a CTLA-4 associated immune dysregulation, a hemaphagocytic syndrome, ALPS syndrome, or a syndrome caused by heterozygous PTEN germline mutations.

In certain embodiments, the T cell mediated disease is immunodysregulation polyendocrinopathy enteropathy X-linked syndrome (IPEX; OMIM http://www.omim.org/entry/304790) or an IPEX-like syndrome and said genomic location is a mutation comprised in a gene selected from the *Foxp3* gene, the CD25 gene, the *Stat5b* gene, the *Stat1* gene and the *Itch* gene (Verbsky and Chatila, Curr Opin Pediatr. 2013 Dec;25(6):708-14). The mutations in said genes prevent the expression or normal function of the gene product. Editing these genomic locations to eliminate the mutation restores gene and protein expression. In certain embodiments, the treatment relates to the treatment of an immunodeficiency, in particular (severe combined immunodeficiency syndrome (SCID).

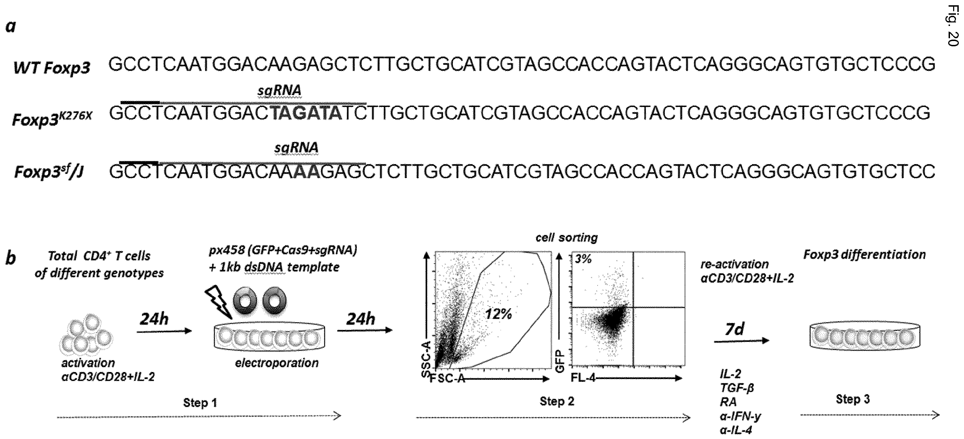

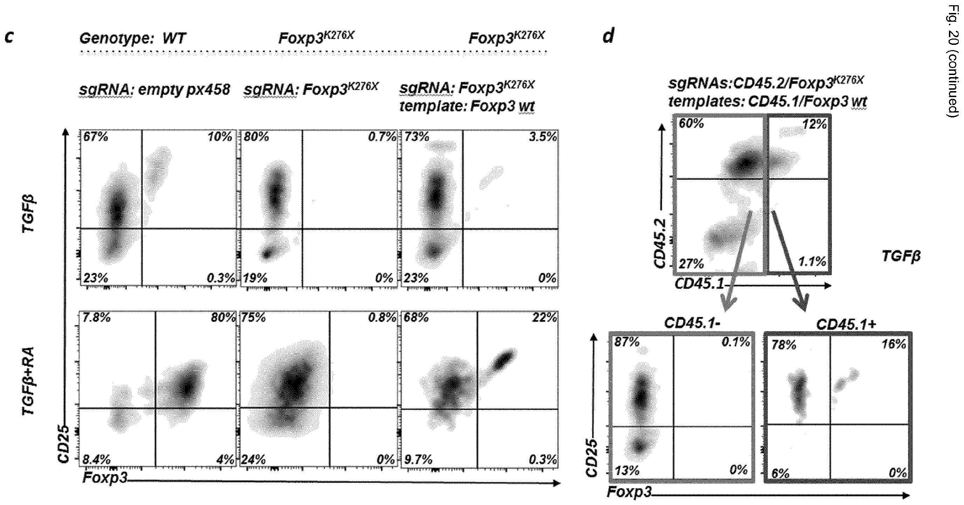

In certain embodiments, the disease-related gene defect is a mutation in the *Foxp3* gene and said disease is immunodysregulation polyendocrinopathy enteropathy X-linked syndrome (IPEX). In certain embodiments, the disease-related gene defect is a mutation in Foxp3, particularly the Foxp3^{K276X} mutation. This mutation prevents the normal function of the gene product. Editing this genomic location reverts the mutation to the wildtype DNA sequence the mutation and thus restores the *Foxp3* gene and Foxp3 protein expression (Fig. 20).

IPEX can be considered a malignant disease comparable to tumor/cancer, since it is very aggressive although it has a different mechanism. Ablation of the pathogenic hematopoietic/immune cells carrying the disease-related gene defect is justified, at least transiently.

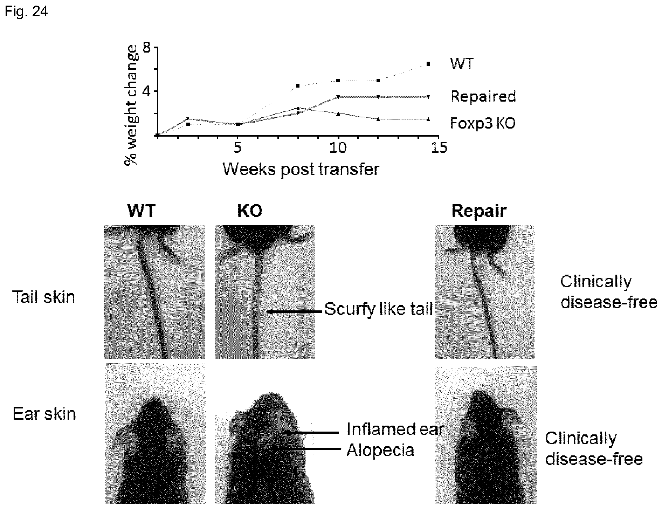

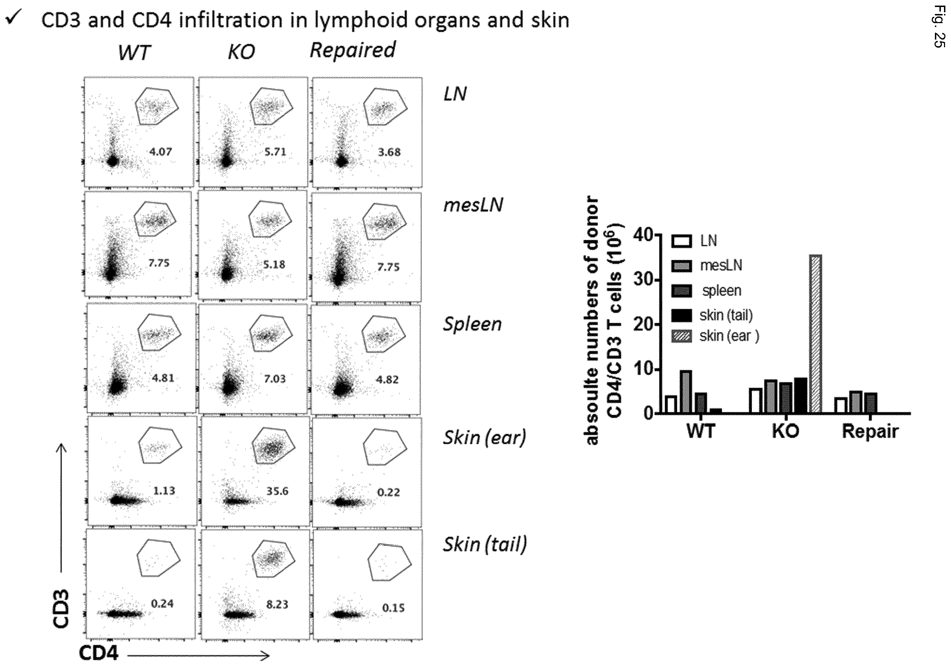

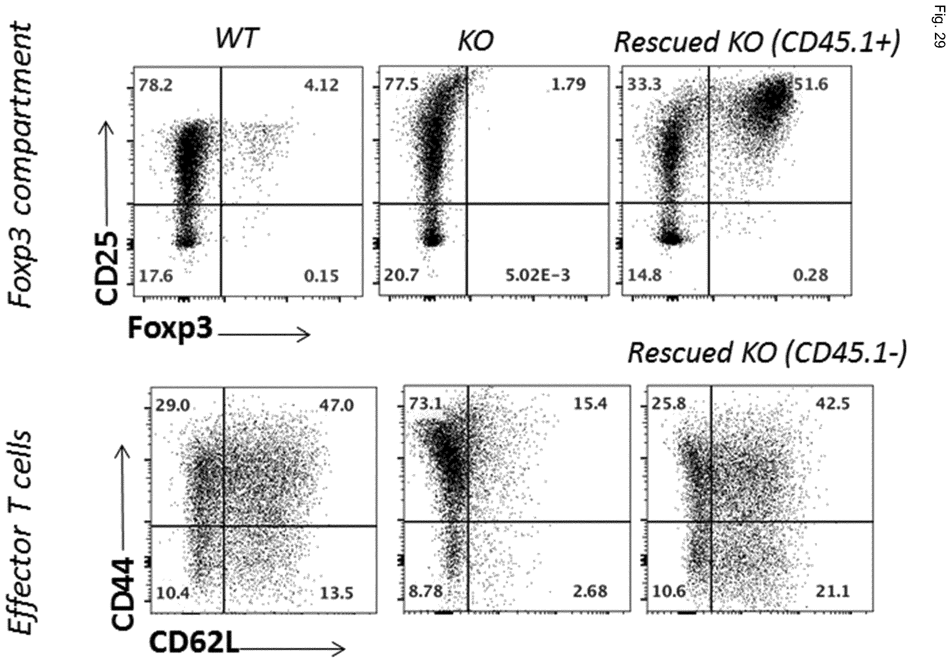

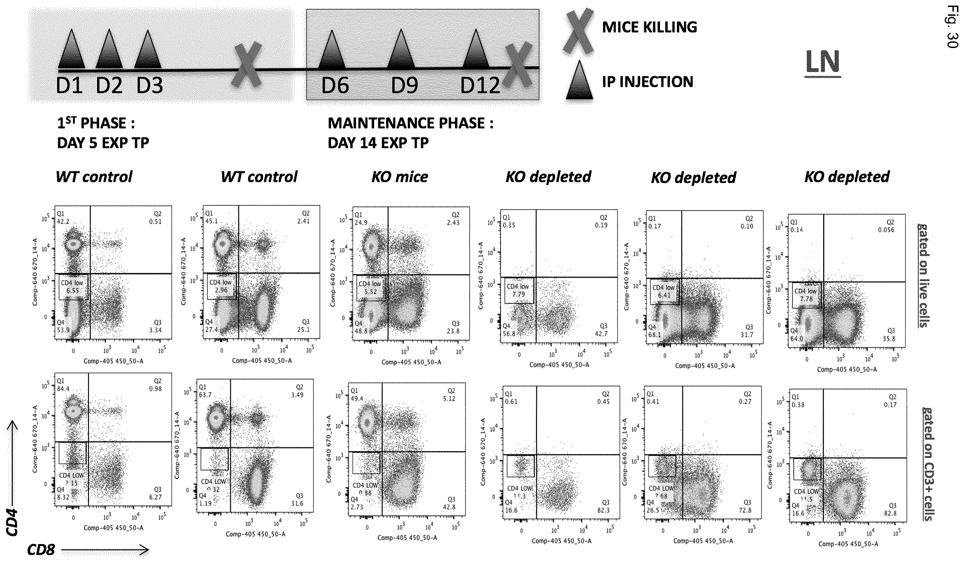

Within the context of the present specification, the term *"Foxp3* gene" relates to human forkhead box P3, NCBI GENE ID: 50943. In the animal experiments described in the examples section, the murine Foxp3^{K276X} mutation was repaired by gene editing. This mutation recapitulates a clinically relevant human Foxp3 mutation (Ramsdell et al., Nature reviews. Immunology 14, 343-349 (2014); Lin et al., The Journal of allergy and clinical immunology 116, 1106-1115 (2005)). The inventors have shown that it is possible to correct the Foxp3^{K276X} mutation in murine T cells using gene editing (Fig. 20, 21, 22, 26). The inventors have further demonstrated that repaired T cells are functional and can suppress other immune cells (Fig. 24, 25). These T cells can either be directly repaired or can be derived from repaired HSCs, or iPS-derived T cells. The inventors further demonstrated that adoptively transferred T cells expand in Foxp3-deficient hosts. Up to half of the T cells become Treg and suppress diseases (Fig. 29). This demonstrates the feasibility of T cell therapy for scurfy/IPEX syndrome. The inventors have shown that CD4 depletion can prevent disease (Fig. 30-32). Thus, CD4+ cells are the pathogenic cells. Therefore, in principle a therapeutic approach could be to deplete CD4+ T cells and then transfer Foxp3-reparied CD4+ T cells to restore immunologic balance. However, since the gene-repaired T cells and the pathogenic Foxp3-deficient T cells both express CD4, continuous depletion of CD4+ T cells will not only kill the pathogenic cells but also the adoptively transferred, gene-repaired cells. A solution would be to simultaneously correct the Foxp3 gene and in parallel do allele engineering. The inventors demonstrate the feasibility of correcting the Foxp3 gene and converting CD45.2 to CD45.1 (Fig. 20). Therefore, the pathogenic Foxp3-deficient cells could be depleted using an antibody directed against the natural CD45 epitope (e.g. CD45.2). If the Foxp3-repaired cells are converted to an engineered CD45 allele (e.g. CD45.1) then the transferred, gene-repaired cells will no longer be depleted. This allows simultaneous depletion of pathogenic cells and replenishment with functional, repaired cells until immunologic balance is restored. At this point the selective ablation of the pathogenic host cells can be stopped. The inventors have shown that CD45 depletion indeed expands survival of Foxp3-deificient mice substantially.

Moreover, Foxp3-gene corrected cells not only reexpress Foxp3 protein but also upregulate CD25, the high affinity Interleukin-2 (IL-2) receptor in vitro and in vivo (Fig. 20, 21, 22, 26). Physiologically, Foxp3 positively regulates CD25 expression in wildtype T cells and the IL-2/CD25/Stat5 axis is required for the survival of regulatory T cells. Therefore the inventors postulated that gene-corrected Foxp3-reexpressing T cells could be expanded in vivo by therapeutic administration of IL-2 (Fig. 23). It is well established that T cells can be expanded in vivo using IL-2/anti-IL-2mAb complexes, low dose IL-2 therapy, (engineered) IL-2 variants, other Treg expansion protocols. Fig. 27 shows that repaired T cells can be expanded in vivo using IL-2/anti-IL-2mAb complexes.

In certain embodiments, the disease-related gene defect is a mutation comprised in the CTLA-4 gene and the disease is human immune dysregulatory syndrome associated with CTLA-4 mutations (Schubert et al., Science Translational Medicine 5, 215ra174-215ra174 (2013); Kuehn et al., Science (New York, N.Y.) 345, 1623-1627 (2014)).

A second aspect of the disclosure provides, but not as part of the claimed invention, an agent selected from
a. a compound comprising, or consisting of, an antibody or antibody-like molecule and
b. an immune effector cell bearing an antibody-like molecule or an immune effector cell bearing a chimeric antigen receptor,
for use in a treatment of a medical condition. The agent is specifically reactive to either a first or a second isoform of a surface protein, wherein the first isoform of the surface protein is functionally indistinguishable, but immunologically distinguishable from the second isoform of the surface protein. The agent is administered to ablate a cell bearing the isoform that the agent is reactive to.

In certain embodiments, the medical condition is a hematopoietic disorder.

In certain embodiments, the medical condition is a malignant hematopoietic disease.

In certain embodiments, the medical condition is a malignant hematopoietic disease refractive to treatment with anti-CD19 CAR T-cells (= CAR19 cells).

In certain embodiments, the medical condition is a non-malignant hematopoietic disease.

In certain embodiments, the medical condition is an autoimmune disease.

In certain embodiments, the medical condition is graft-versus-host disease (GvHD).

In the context of the present specification, graft-versus-host disease relates to a medical complication following the receipt of transplanted tissue from a genetically different person. Immune cells in the donated tissue (the graft) recognize the recipient (the host) as foreign.

In certain embodiments, the medical condition is graft-versus-host disease caused by hematopoietic stem cell transplantation.

In certain embodiments, the medical condition is graft-versus-host disease caused by adoptive transfer. In the context of the present specification, adoptive transfer or adoptive cell therapy relates to the transfer of human cells, usually immune cells, into a patient. The cells may be autologous or allogeneic. Targeted modifications effected by gene editing techniques allow to customize the transferred cell product to repair genetic defects, increase the efficiency of the transferred cells or equip the cells with additional desired features such as guidance molecules or safety switches.

In certain embodiments, the medical condition is graft-versus-host disease caused by organ transplantation.

In certain embodiments, the antibody or antibody-like molecule is coupled to a toxin, thereby forming an immunotoxin. In certain embodiments, the antibody or antibody-like molecule is coupled to saporin.

In certain embodiments, the agent is a bispecific antibody or bispecific antibody-like molecule. In other words, the agent is an antibody or antibody-like molecule that can simultaneously bind to two different types of antigens.

In certain embodiments of the present disclosure, the agent is an immune effector cell bearing a bispecific antibody or bispecific antibody-like molecule.

In certain embodiments, the agent is an immune effector cell bearing
a. a first antibody or antibody-like molecule specifically reactive to either a first or a second isoform of a first surface protein, wherein said first and said second isoform of said first surface protein are functionally indistinguishable, but immunologically distinguishable, and
b. a second antibody or antibody-like molecule specifically reactive to either a first or a second isoform of a second surface protein, wherein said first and said second isoform of said second surface protein are functionally indistinguishable, but immunologically distinguishable.

In certain embodiments, the first surface protein is CD19.

In certain embodiments, the second surface protein is CD45 or CD34.

In certain embodiments, the first surface protein is CD19 and the second surface protein is CD45.

In certain embodiments of the present disclosure, the agent is an immune effector cell bearing an antibody or antibody-like molecule, or an immune effector cell, in particular a T cell, bearing a chimeric antigen receptor, for use in a method of treatment of a malignant hematopoietic disease.

In certain embodiments of the present disclosure, the agent is a T cell bearing a chimeric antigen receptor directed against CD45 for use in a method of treatment of a hematopoietic disease, in particular a malignant hematopoietic disease.

CD45 is a particularly good target for the treatment of hematopoietic disease as it is expressed on all hematopoietic cells including malignant cells. CD45 critical for cell survival, therefore cells will have more difficulties to develop resistance.

Another aspect of the disclosure provides, but not as part of the claimed invention, a combination medicament comprising
a. a first agent according to the second aspect of the disclosure, wherein the agent is reactive to a first surface protein, and
b. a second agent according to the second aspect of the disclosure, wherein the agent is reactive to a second surface protein.

In certain embodiments, the combination medicament is provided for treatment of a hematopoietic disease.

In certain embodiments, the combination medicament is provided for treatment of a malignant hematopoietic disease.

In certain embodiments, the combination medicament is provided for treatment of a non-malignant hematopoietic disease.

In certain embodiments of the present disclosure, the first and the second agent are T cells bearing a chimeric antigen receptor.

In certain embodiments, the first surface protein is CD19 and the second surface protein is CD45.

In certain embodiments of the present disclosure, the first agent is an anti-CD19 CAR T-cell and the second agent is an anti-CD45 CAR T-cell.

In certain embodiments, the malignant hematopoietic disease is refractive to treatment with anti-CD19 CAR T-cells (= CAR19 cells) alone.

Another aspect of the disclosure provides, but not as part of the claimed invention, a method for in vivo tracking of a cell expressing a first isoform of a surface protein, wherein said first isoform of said surface protein is functionally indistinguishable, but immunologically distinguishable from a second isoform of said surface protein, said method comprising administrating to said patient a ligand specifically reactive to said first isoform.

An alternative of this aspect of the disclosure provides, but not as part of the claimed invention, a method for tracking of a cell expressing a first isoform of a surface protein in a tissue derived from a patient, wherein said first isoform of said surface protein is functionally indistinguishable, but immunologically distinguishable from a second isoform of said surface protein, said method comprising administrating to said tissue derived from said patient a ligand specifically reactive to said first isoform.

In certain embodiments of this aspect of the disclosure the tissue derived from a patient relates to a blood sample. Such a blood sample may be analyzed usng FACS. In certain embodiments of this aspect of the disclosure, the tissue derived from a patient relates to a tissue sample of an organ, e.g. a biopsy, e.g. a liver sample. Such a tissue sample may be analyzed using histological methods.

Yet another aspect of the disclosure provides, but not as part of the claimed invention, a method for selectively depleting or enriching a cell in vivo, comprising the steps of
a. providing a cell, wherein said cell expresses a first isoform of a surface protein, which is different from a second isoform of said surface protein with regard to an amino acid marker, wherein said first isoform comprises amino acid marker A encoded by nucleic acid sequence A, and said second isoform comprises amino acid marker B encoded by nucleic acid sequence B;
b. inducing a mutation from said nucleic acid sequence A to said nucelic acid sequence B in the genomic DNA of said cell;
c. selectively enriching/depleting said cell based on the expression of said first or said second isoform of said surface protein.

In the context of the present specification, the term "selective depletion of cells" relates to selectively reducing the total number or concentration of cells expressing a certain marker/allele/isoform.

The skilled person is aware that in instances where a defined volume comprises cells expressing a first isoform and cells expressing a second isoform, the selective depletion of cells expressing the first isoform corresponds to enrichment of cells expressing the second isoform. By way of non-limiting example, selective depletion can be achieved by complement-dependent cytotoxicity (CDC), Antibody-dependent cellular cytotoxicity (ADCC), or Antibody-drug conjugate (ADC). Selective depletion can also be effected by administration of an antibody or antibody-like molecule that is not coupled to an effector compound such as a drug or a toxin.

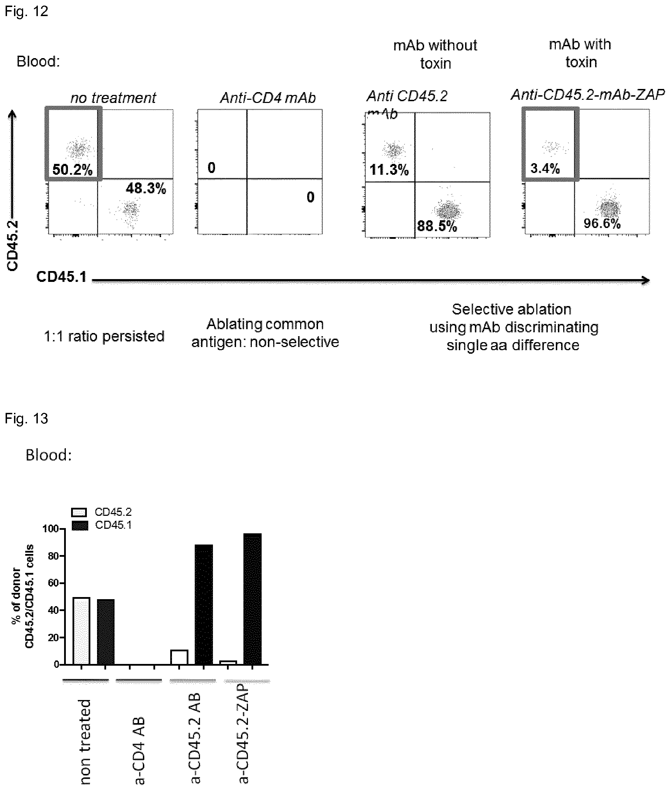

The inventors have demonstrated that selective in vivo depletion is possible using antibodies against CD45.2 or CD45.1 , respectively (Fig. 12). Using an antibody directed against an epitope shared by two cell populations (e.g. CD45.2+ and CD45.1+ T cells) results in non-selective depletion of both cell populations (e.g. anti-CD4 depletion). In contrast, depleting with an antibody which selectively binds to one (e.g. CD45.2) but not the other (e.g. CD45.1) allele of a commonly expressed surface protein (e.g. CD45) only depletes the cells expressing the allele bound by the specific mAb. In the example shown depleting CD45.2+ cells spares CD45.1+ cells and therefore results in a relative enrichment of CD45.1+ cells. Depletion can be more efficient if a toxin is coupled to the mAb.

An advantage of using an antibody or antibody-like molecule that is not coupled to a toxin can be that treatment using an immunotoxin leads to HSC depletion while treatment using an antibody or antibody-like molecule not coupled to a toxin spares HSC. This can be desired because the hematopoietic system is partly retained.

The inventors have demonstrated that a single amino acid difference can be engineered into a cell and can be discriminated by two different ligands that specifically bind to the two isoforms/alleles (native vs. engineered). A specifically designed artificial mutation or a rare but naturally occurring mutation such as a single nucleotide polymorphism (SNP) is engineered into an endogenous surface expressed gene to change its antigenicity. The skilled person is aware that this mutation may be introduced by any method known in the field, including HDR and base editors. This altered epitope is subsequently exploited to selectively deplete successfully edited cells with a ligand which specifically and selectively recognizes this artificial epitope. Alternatively, the edited cells are rendered resistant to depletion by a ligand which recognizes the natural epitope (and hence can deplete host cells) but does not recognize the altered epitope and therefore spares the transferred cells.

In instances where the "edited/engineered cells" (cells in which the first isoform of the cell surface protein has been changed to the second isoform) are subsequently used for transplantation, in particular adoptive transfer, the two different isoforms can be used to discriminate between transferred cells and host cells. This enables tracking of the transferred cells since they are permanently marked. Tracking can be achieved with labelled ligands either in vivo or ex vivo e.g. by flow cytometry or histochemistry on cells or tissues. In vivo application of ligands specific for either the transferred cells or the host cells enables selectively depleting either the transferred cells or the host cells using the antibody that only binds to the transferred, engineered cells or the host cells, respectively. Selective cell depletion can also be achieved by cells carrying a natural or a chimeric antigen receptor (CAR) recognizing either the transferred cells or the host cells.

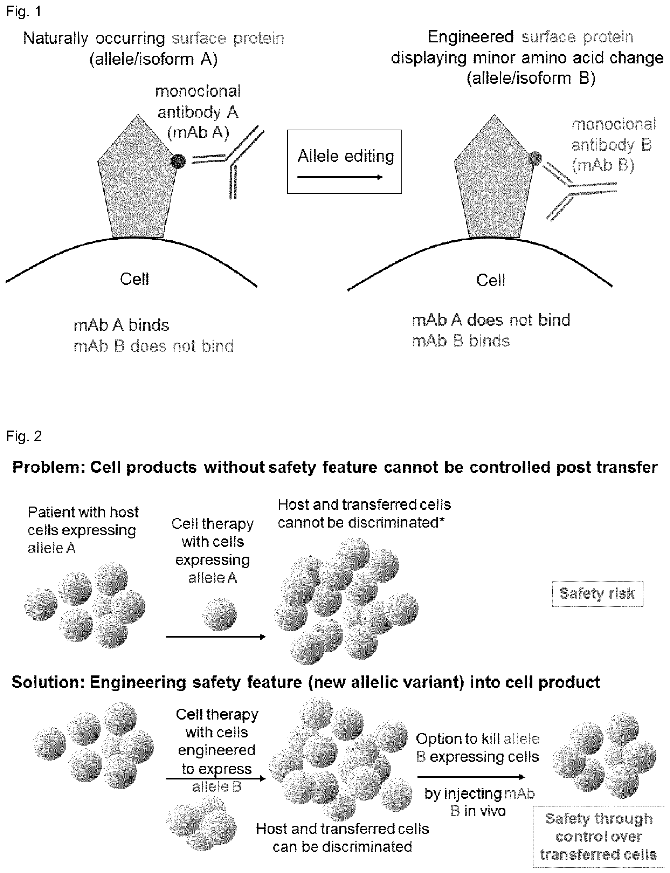

Selective depletion of the engineered cells constitutes an important safety feature by providing a "safety or kill switch". The basic concept of safety switches and suicide genes is described in Jones et al., Front Pharmacol.; 5:254. doi: 10.3389. The approach of the inventors is simpler, safer and more versatile. In principle any cell which is adoptively transferred can be engineered to carry the altered allele/epitope as a combined in vitro or in vivo selection, tracking, safety and/or selective ablation switch. Non-exclusive examples include cells which only carry the engineered allele but are otherwise not genetically engineered or cells which carry additionally engineered features such as CAR cells. For instance, transferred allogeneic cells which are used for their graft-vs-leukemia effect can cause graft-vs-host disease (GvHD). If the engineered allele is incorporated before transfer they can be eliminated by the engineered allele to reduce/treat GvHD (Fig. 2). Similarly, transferred autologous tumor infiltrating lymphocytes (TILs) or pathogen-specific lymphocytes can be engineered to carry the altered allele to eliminate them if unwanted side-effects occur due to off-target effects or too intense on-target effects (Fig. 2). CARs can be derived from mAb to combine the specificity of a known mAb with the features of a cell, e.g. a killer T cell. The principle of redirecting killing (or suppressive) activity of a given (T) cell to a specific target antigen by introducing a CAR is in principle applicable to a wide array of diseases, including malignancies but also autoimmune diseases, transplantation or other hematopoietic diseases. The success of CAR19 T cells demonstrates the potential of CAR cells as therapeutic agents. Importantly however, while CAR19 T cells are highly efficacious to cure some CD19+ tumors, several CAR T cells reactive against a variety of different target molecules can have severe, at times fatal side-efects such as cytokine release syndrome and/or neurotoxicity (demonstrated for several CAR constructs targeting CD19 but also other targets including but not limited to CD123). Therefore, the ability to control/eliminate CAR T cells after transfer is important (Fig. 2, 10, 19). In the case of CAR cells the altered allele can serve as a safety switch. Moreover, transferred, engineered cells can also be eliminated in case they become malignant or cause any type of unwanted on-target or off-target damage (possibly years later). Alternatively, disease causing host cells can be selectively ablated while sparing autologous but engineered cells. In contrast to the inventors' method, existing technology is restricted to ablation of the transferred cells but does not easily allow ablation of host cells. The altered isoform allows to transfer e.g. gene-repaired or otherwise engineered autologous cells during ablation of host cells. Without the isoform switch introduced by the method of the disclosure, the host cell ablation needs to be stopped when the healthy cells are transferred. In this case, while the newly transferred, repaired cells expand the host cells will also expand and can no longer be ablated, risking that the disease-causing host cells will outcompete the repaired cells. Therefore rendering the engineered cells resistant to depletion by the method of the disclosure is highly relevant as a therapeutic approach. As an example, the CD19 epitope recognized by anti-CD19-CAR cells could be mutated in autologous hematopoietic cells such that depleting anti-CD19 mAb or anti-CD19-CAR cells no longer can bind and destroy the engineered cells but CD19 would remain functional. This would eliminate a major complication of today's effective anti-CD19-CAR cells. While anti-CD19-CAR have very high success rates eliminating CD19 expressing hematopoietic malignomas they concordantly lead to eliminating of CD19 expressing healthy host cells. This leads to hypogammaglobulinemia and therefore increased risk for infections. A mutated CD19 would allow reconstitution of the host immune system with healthy autologous hematopoietic stem cells (HSCs), which will give rise to B cells which are resistant to the anti-CD19-CAR cells. The CAR 19 T cells might therefore continuously prevent a relapse while the edited resistant cells will provide natural protection from infections. Patients would therefore no longer depend on IVIG infusions. HSC transplantation could potentially be achieved as partial chimerism through non-genotoxic pre-conditioning, e.g. through antibodies (Nat Biotech, 2016). Alternatively, anti-CD45-CAR cells recognizing an epitope found in the common human population (e.g. analogous to CD45. 2) could be used to eliminate all hematopoietic host cells including malignant or otherwise disease-causing hematopoietic cells. CD45 is a good target since it is expressed on all hematopoietc cells including most malignant cells. Furthermore, CD45 is critical for survival of lymphocytes, therefore if targeted by CAR T cells it is less likely that cells can downregulate CD45 or mutate CD45 to escape targeting by CAR T cells. This would reduce the risk for relapse. Transplantation of healthy autologous hematopoietic stem cells (HSCs) or other hematopoietic cells carrying an engineered CD45 epitope (e.g. CD45.1) as illustrated by the CD45.2 to CD45.1 switch experiments would allow to reconstitute the host with a healthy hematopoietic system which will no longer be depleted by the anti-CD45-CAR cells. A major advantage would be that all CD45 expressing malignancies (including but not restricted to T cell and myeloid malignancies) can be targeted without the need for tumor- or cell type-specific antigens, i.e. the disclosure would provide a universally applicable system to treat hematopoietic malignancies and other non-malignant hematopoietic diseases. This would overcome a major hurdle of CAR-T therapy: identifying appropriate target antigens (Klebanoff, Nat Med 2016). It could therefore substantially expand the disease indications which are amenable to CAR-T therapy. Although CAR-T therapy is highly successful treating CD19+ tumors, other hematopoietic malignancies pose major challenges. As an example, treating multiple myeloma remains a major challenge, in part due to the lack of a suitable target antigen (Mikkilineni, Blood, 2017; Sadelain, Nature 2017). In addition, hematopoietic tumors could be treated without the need for allogeneic cells therefore eliminating GvHD as a major complication. Moreover, reconstitution can start during the depletion phase, which will shorten time to recovery. Importantly, the mutation used to render the transferred cells resistant to depletion can later also be used to deplete those cells again should this become necessary. CAR cell dependent depletion of HSCs could potentially be used as an alternative way of achieving mild, i.e. non-genotoxic preconditioning. CAR45 cells will also eliminate HSCs. The currently used "hit and replace" strategy employed for "bone marrow" or HSC transplantation could be achieved using CAR45. Instead of using toxic chemotherapy and irradiation, preconditioning of the patient could be achieved with CAR45. An advantage of removing HSCs and the hematopoietic system using CAR45 cells and replacing it with allele engineered, resistant HSCs would be that the dangerous post-transplant time might be eliminated since hematopoietic reconstitution can start during the ablation of the unwanted cells. Thus, the patients would always have a functional immune system rather than go through a prolonged phase of bone marrow depletion and immunosuppression. Thus, the inventor's strategy could eliminate infections as major complications of current HSC transplantation. CAR cells directed against an antigen or a combination of antigens to restrict the target cells specifically to HSCs could be used to deplete endogenous HSCs. This could be e.g. anti-CD45 or anti-CD34 plus a second antigen in a synthetic biology approach (e.g. with an AND gate) to specifically and exclusively direct the CAR cells against HSCs.

CAR45 treatment combined with replacement of the hematopoietic system with allele engineered HSCs could be used as an alternative to CAR19 therapy (since all CD19+ cells also express CD45) or it could be used as a combination therapy of CAR19 plus CAR45. CAR45 therapy could also be used for relapsed CD19negative or CD19 mutated malignomas after CAR19 therapy.

This aspect of the disclosure represents a universal strategy to replace cells. The cells may be hematopoietic cells, autologous or allogeneic. If the replacing cells are HSCs, the described method can be used to treat any hematopoietic malignancy or other hematopoietic disorders. Other advantages of the approach of the inventors compared to existing "safety switch" approaches include the following. The inventors' approach uses an endogenous protein. No transgene or tag has to be introduced into the cell. The two epitopes are functionally identical, but can be distinguished by specifically binding ligands. The approach enables both depletion of transferred cells or host cells, depending which ligand is used. Since the designed mutation is introduced into the genome the safety feature remains permanently in the cells and will not get silenced which can happen to virally introduced transgenic safety switches. In addition, the engineered epitope will be less antigenic than artificial large safety switch/suicide gene constructs and will therefore less likely be rejected by host cells. Moreover, the use of engineered isoforms relies on targeted mutations and is therefore likely safer than other safety switches/suicide genes which are randomly integrated into the genome, usually by viral delivery and can therefore lead to insertional mutagenesis (Cornu, Nat Med, 2017).

The skilled person is aware that in order to change the cell surface protein from the first isoform to the second isoform, alternative methods can be applied instead of HDR. By way of non-limiting example, the isoform switch can be effected using base editors as described in the following publication: Komor et al., Nature 533, 420-424, doi:10.1038/nature17946. This approach could increase the safety even further by allowing editing of the desired amino acid without the need for a dsDNA break. Base editors or related technologies can be delivered as plasmids or minicircles (dsDNA), mRNA or RNP.

In instances where the switching of a first isoform of a cell surface protein to a second isoform is combined with the repair of a disease-causing gene (e.g. the Foxp3 gene), it is possible to deplete the non-repaired cells in vivo (i.e. after transfer into the host) by depleting the cells expressing the first isoform. The inventors have demonstrated that in instances where Isoform switch and gene defect repair are both effected by HDR, the likelihood of a successfully repaired gene is increased in cells in which the isoform switch has occurred (Fig. 20). Combining an isoform switch at a first gene with a genetic modification at a second gene allows to include a safety feature into genetically engineered cells.

The isoform switch can also be employed as a marker to trace edited, transferred cells in a host.

According to an alternative aspect, a method for selectively depleting or enriching a cell in a composition of non-edited and edited cells is disclosed, but not as part of the claimed invention, wherein the method comprises the steps of
a. providing a cell, wherein the cell expresses a first isoform of a surface protein, which is different from a second isoform of the surface protein with regard to an amino acid marker, wherein the first isoform comprises amino acid marker A encoded by nucleic acid sequence A, and the second isoform comprises amino acid marker B encoded by nucleic acid sequence B;
b. inducing in said cell by site specific genetic manipulation the exchange of nucleic acid sequence A to nucleic acid sequence B, thereby changing in said cell the expression of the first isoform to the expression of the second isoform;
c. selectively enriching/depleting the cell based on the expression of the first or the second isoform of the surface protein.

In certain embodiments, the genetic manipulation resulting in said insertion, deletion and/or substitution of amino acids is effected by homology directed repair following a double strand break induced by a CRISPR-associated endonuclease (Cas9) and a guide RNA, wherein said guide RNA is capable of annealing to said first genomic location.

In the context of the present specification, "CRISPR-associated endonuclease" refers to a Cas9 endonuclease known in the art to facilitate CRISPR-like sequence-guided cleavage of DNA strands. Non-limiting examples of a CRISPR-associated endonuclease are the Cas9 endonucleases of Streptococcus pyogenes (SpyCas9), the Cpf1 endonuclease of Francisella (FnCpf1), Acidaminococcus (AsCpf1) and Lachnospiraceae bacterium (LbCpf1), to any orthologues of SpyCas9, FnCpf1, AsCpf1 or LbCpf1, or to any engineered protein variants of SpyCas9, FnCpf1, AsCpf1 or LbCpf1 or their orthologues. The skilled person is aware that the disclosure also encompasses newly discovered or engineered CRISPR/Cas variants.

In the context of the present specification, the term "orthologue" refers to a gene and its corresponding polypeptide that evolved by vertical descent from a single ancestral gene. In other words, orthologues genes/polypeptides share a common ancestor and were divided when a species diverged into two separate species. The copies of a single gene in the two resulting species are then referred to as orthologues. To ascertain that two genes are orthologues a person skilled in the art can carry out a phylogenetic analysis of the gene lineage by comparing the aligned nucleotide or amino acid sequences of genes or polypeptides.

In the context of the present specification, the term "guide RNA" refers to a synthetic RNA able to guide a CRISPR-associated endonuclease to a genomic location of interest (where the endonuclease will cleave a phosphodiester bond within the genomic DNA). The skilled person is aware that if a Cas9 endonuclease is used, the expression "guide RNA" may refer to a single guide RNA (sgRNA) comprising both a sequence necessary for Cas9-binding and a user-defined "targeting sequence", or to a combination of two RNA molecules, wherein one comprises the sequence necessary for Cas9-binding (tracrRNA) and the other comprises the user-defined "targeting sequence" (crRNA). If a Cpf1 endonuclease is used, the expression "guide RNA" refers to a single RNA molecule comprising both the sequence necessary for Cpf1-binding and the user-defined "targeting sequence" or several guide RNAs transcribed as a single crRNA array (Zetsche, Nat Biotech, 2016). The "targeting sequence" is able to anneal to the genomic location of interest and thus defines the genomic target to be modified and usually comprises approximately 20 nucleotides.

DNA cleavage by Cas9 is dependent on the presence of a short protospacer adjacent motif (PAM) in the target DNA, restricting the choice of targetable sequences. CAS9 from Streptococcus pyogenes (SpyCas9) for example corresponds to the PAM sequence 5'-NGG-3'. In certain embodiments, the DNA repair construct comprises a mutated PAM sequence. The mutation renders the PAM sequence non-functional but does not affect protein expression, stability or function. The use of a DNA repair construct comprising a mutated PAM sequence enhances HDR efficiency.

The skilled person is aware that besides the CRISPR system, alternative means for site specific DNA editing exist, namely the use of Zinc finger endonucleases, transcription activator-like effector nucleases (TALEN), meganucleases or argonaute-based systems (Nat Biotechnol. 2016 Jul;34(7):768-73) or base editors (Komor et al., Nature 533, 420-424, doi:10.1038/nature17946). The disclosure also encompasses the use of those alternative means for site specific DNA editing.

In certain embodiments, the first DNA repair construct is not a substrate for the CRISPR system employed in the first step of the method (introducing a strand break into the genomic DNA), because it does not comprise a PAM sequence. Thereby, the inserted sequence can no longer be cut after insertion by a second endonuclease event.

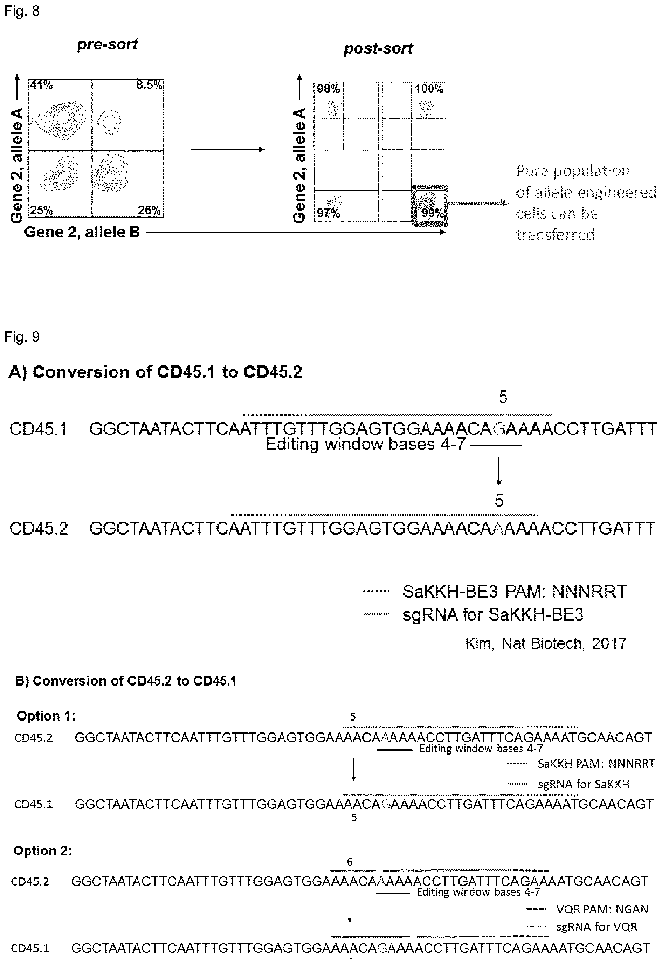

In certain embodiments, the genetic manipulation resulting in said insertion, deletion and/or substitution of amino acids is effected by providing, in particular transfecting/electroporating said cell with, a base editor (as described in Komor et al., Nature 533, 420-424, doi:10.1038/nature17946 and/or Gaudelli, Nature 2017) capable of changing nucleic acid sequence A, encoding amino acid marker A, to nucleic acid sequence B, encoding amino acid marker B, and a guide RNA capable of directing said base editor to nucleic acid sequence A, encoding amino acid marker A. The design of a base editor capable of changing murine CD45.1 to CD45.2 is illustrated in the examples section. (Fig. 9). This illustrates the feasibility of using base editors for allele engineering as an alternative to the proven HDR approach. However, due to limitations as to which nucleotides in the genome are amenable to base editing, the inventors were unable to design base editors to convert CD90.2 to CD90.1 or CD90.1 to CD90.2. In addition, based on the original base editor design relying on the NGG PAM (as described by Komor et al., Nature 2016), it was not possible to design a base editor to convert either CD45.1 to CD45.2 or CD45.2 to CD45.1. In contrast, the generation of engineered Cas9 variants recognizing altered PAM site specificity expanded the number of nucleotides that can be targeted by base editors (Kim, Nat Biotech 2017). The inventors identified a PAM site for the Staphylococcus aureus (SaKKH-BE3) in the proximity of the G present in the CD45.1 sequence which when mutated to an A results in the CD45.2 allele. They designed a sgRNA which places the G to be converted to an A at the position 5 in the editing window because SaKKH-BE3 most effectively edits a G at position 5 over other positions in the editing window. Next, the inventors designed guideRNA/base editor pairs to convert CD45.2 to CD45.1 (Figure 9b). The newly engineered A/T adenince base editors (ABE) can convert G/C. With this new ABE it is in principle possible to convert the necessary A in the CD45.2 allele to a G in the CD45.1 allele. However, the currently available ABEs require a NGG PAM in the vicinity of the desired mutation but there is no such PAM to correctly position the ABE. Therefore the inventors designed a hypothetical ABE based on the rules applicable for cytidine deaminase base editors. Since the known cytidine deaminase base editors and the new ABEs edit in a similar editing window it is reasonable to assume that this strategy will work. The inventors identified 2 options, one based on a hypothetical Cas9 SaKKH-ABE fusion and one based on a hypothetical Cas9 VQR-ABE fusion (Fig. 9b). It cannot be excluded that flanking As will also be converted which might change the epitope. Thus, converting CD45.2 to CD45.1 with base editors will require highly specific base editors with a narrow editing window. Recently reported base editors have editing windows of 1-2 nucleotides and are therefore suitable (Kim, Nat Biotech, 2017). Collectively, these examples illustrate that the principle of converting a base for allele editing is platform independent and can be achieved by HDR-mediated approaches or alternatives such as base editing. Depending on the cell type and genomic context of a desired mutation one or the other may be chosen.

In the context of the present specification, a "DNA repair construct" refers to a DNA construct that is used as a template to repair a DNA strand lesion, particularly a double strand break (DSB), within the genomic DNA by HDR. A DNA repair construct comprises homology arms and a transgenic sequence of interest. The homology arms are homologous to the genomic DNA sequences 5' and 3' of the DSB. The transgenic sequence of interest is located between the homology arms. During genomic DNA repair by HDR, the transgenic sequence of interest is inserted into the genomic DNA. The skilled person is aware that the DNA repair construct can be linear (single stranded or double stranded) or circular (e.g. plasmid, minicircle plasmid).

The skilled person is aware that the expression "the guide RNA is capable of annealing to the genomic location of interest" refers to the fact that part of the guide RNA (the user-defined "targeting sequence") is capable of annealing to the genomic location of interest under high stringency conditions. The guide RNA comprises other parts that are not capable of annealing to the genomic location of interest. By (partly) annealing to the genomic location of interest, the guide RNA directs the CRISPR-associated endonuclease to the genomic location of interest, thereby effecting a DSB at the genomic location of interest.

In certain embodiments, HDR enhancing reagents, in particular vanillin or rucaparib, are used during the gene editing protocol. Within the context of the present specification, vanillin refers to 4-Hydroxy-3-methoxybenzaldehyde, CAS No. 121-33-5. Within the context of the present specification, rucaparib refers to 8-Fluoro-2-{4-[(methylamino)methyl]phenyl}-1,3,4,5-tetrahydro-6H-azepino[5,4,3-cd]indol-6-one, CAS No. 283173-50-2.

Wherever alternatives for single separable features are laid out herein as "embodiments", it is to be understood that such alternatives may be combined freely to form discrete embodiments of the disclosure disclosed herein.

The disclosure is further illustrated by the following examples and figures, from which further embodiments and advantages can be drawn. These examples are meant to illustrate the disclosure but not to limit its scope.

### Brief description of the figures

Fig. 1 illustrates the principle of engineering immunologically discernible variants of cell surface proteins. Isoform A and B are capable of performing the same function (are functionally comparable). In this example isoform A represents a naturally occurring surface protein that is engineered into variant isoform B.
Fig. 2 illustrates how immunologically discernible cell surface variants increase the safety of human cell therapy.

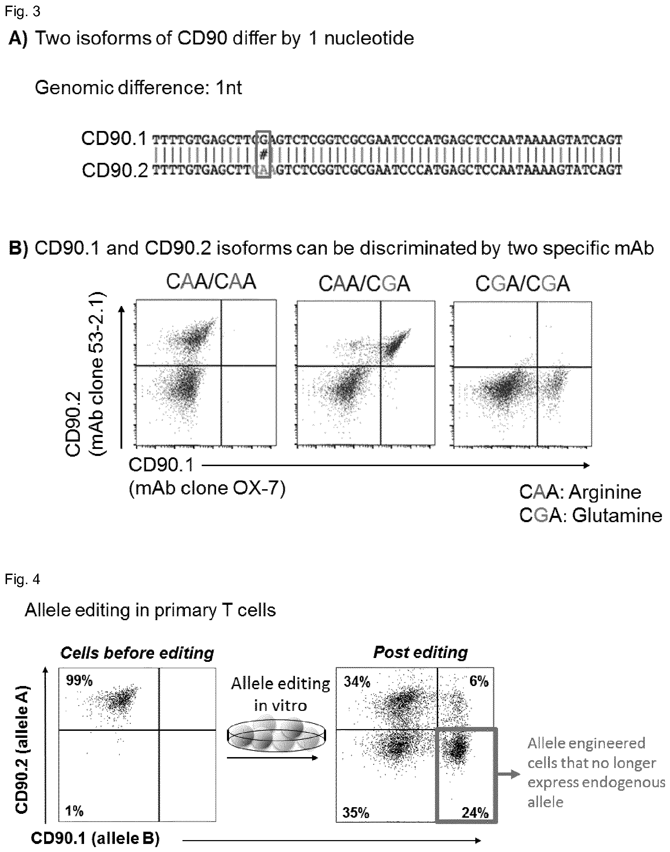

Fig. 3 shows that two isoforms of a surface protein can be distinguished by monoclonal antibodies (mAb). Sequences: upper row: SEQ ID NO 020, lower row: SEQ ID NO 021.
Fig. 4 shows a proof-of-concept experiment: Conversion of CD90.2 (allele A) to CD90.1 (allele B) in primary cells. The left flow cytometry panel shows a pure starting population of CD90.2+ primary T cells. Cells are then engineered in vitro to convert CD90.2 to CD90.1. Post editing 4 distinct populations can be discriminated: CD90.2+ homozygous cells (unedited starting population, upper left), CD90.1+/CD90.2+ heterozygous cells (upper right), CD90.1+ homozygous cells (lower right) and cells which lost CD90 (lower left). The allele engineered cells in the bottom right square no longer express the starting/original endogenous allele (CD90.2). A pure population of CD90.1+CD90.2- cells can be used to isolate correctly edited cells in vitro before transfer to a patient.

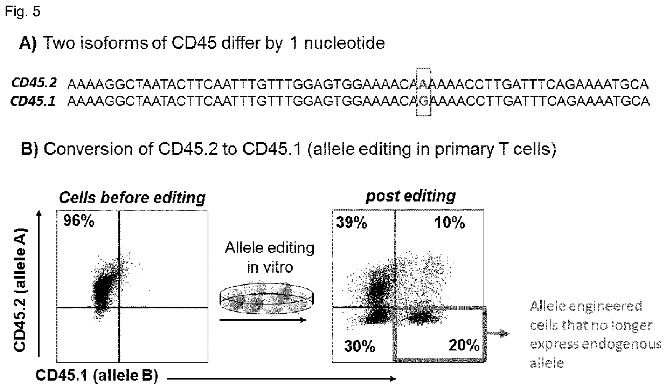

Fig. 5 shows a second proof-of-concept experiment: Conversion of CD45.2 (allele A) to CD45.1 (allele B) in primary cells. Sequences: upper row: SEQ ID NO 022, lower row: SEQ ID NO 023. As shown for CD90.2 to CD90.1 allele conversion (Fig. 4), the allele engineered cells in the bottom right square no longer express the starting/original endogenous allele (CD45.2). This can be used to isolate a pure population of correctly edited cells before transfer to a patient.

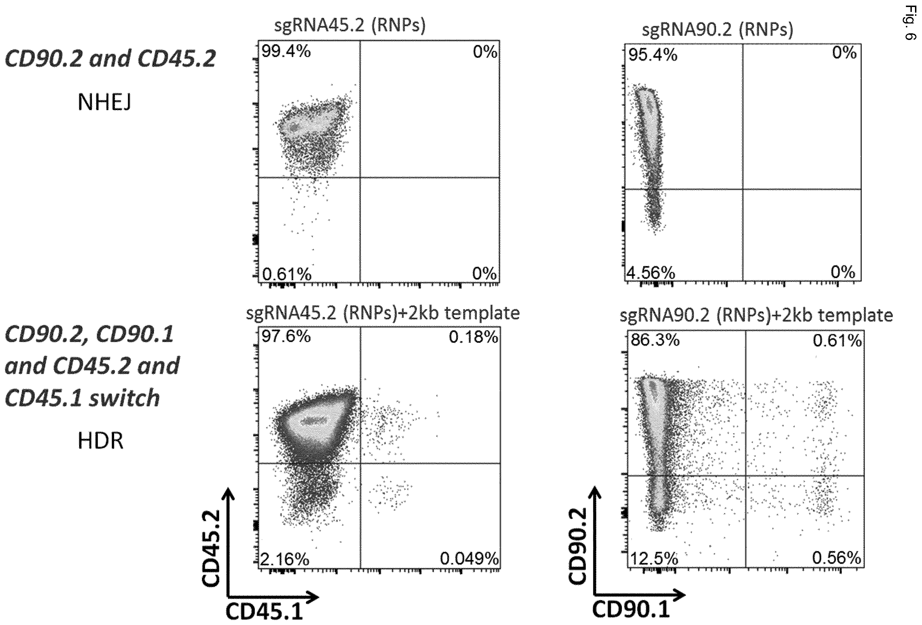

Fig. 6 shows gene editing in EL4 cells using Cas9 ribonucleoprotein particles (RNPs). EL4 cells were transfected with crRNA:tracrRNA/Cas9 complex and +/- HDR 2kb template in the same way as for the plasmid based approach, except for electroporation conditions (described in methods section). This demonstrates that allele editing can be achieved by different approaches, e.g. plasmid-based or RNPs, i.e. is platform independent.

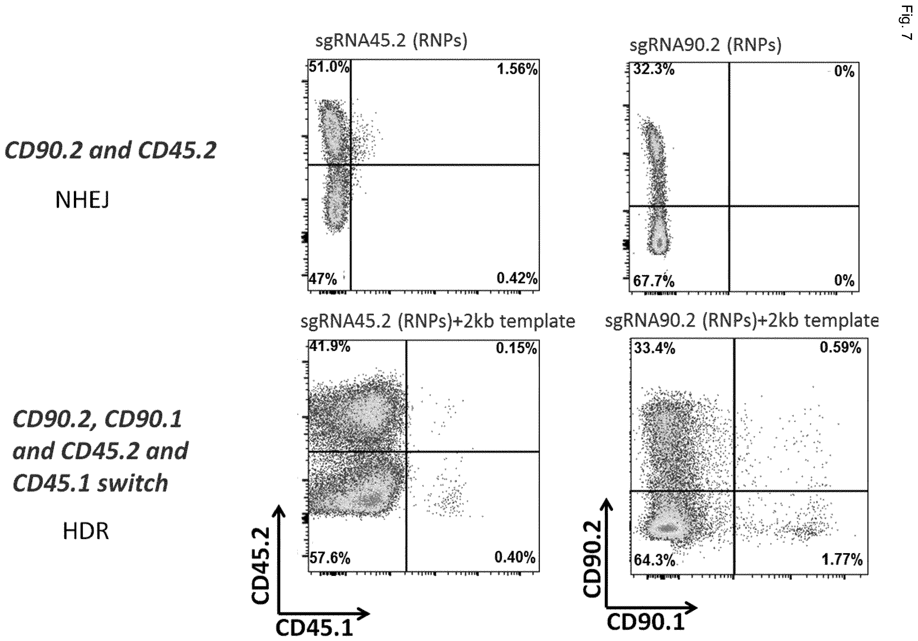

Fig. 7 shows gene editing in primary mouse T cells using Cas9 ribonucleoprotein particles (RNPs). Primary mouse T cells were transfected with crRNA:tracrRNA/Cas9 complex and +/-HDR 2kb template in the same way as for the plasmid based approach. Like Fig. 6 this demonstrates that allele editing to convert allele A to allele B is platform independent.
Fig. 8 shows highly pure ex vivo selection of allele engineered cells. Left panel: After engineering CD45.2 cells into CD45.1 cells 4 population arise (comparable to Fig. 4). Flow cytometry was then used to purify all 4 populations to very high purity. The right panel displays the post-sort purity for each of the 4 populations. Conclusion: All 4 possible cell populations can be purified ex vivo to high purity based on the engineered allele. Correct gene editing was validated by Sanger DNA sequencing (not shown).
Fig. 9 shows the design of a base editor to convert CD45.1 to CD45.2 (A) and CD45.2 to CD45.1 (B). Sequences (A): upper row: SEQ ID NO 024, lower row: SEQ ID NO 025 (B): upper row (option 1 and 2): SEQ ID NO 026, lower row (option 1 and 2): SEQ ID NO 027. A) A guide RNA was designed to be used with the NNNRRT PAM restricted SaKKH-BE3 base editor. The chosen PAM sequence is highlighted by a dotted black bar (ATTTGT). The guide RNA is highlighted by a gray bar, the base editing window of guide RNA nucleotides 4-7 is highlighted. The G at position 5 will be converted to an A with this base editor. Thus, the CD45.1 allele will be converted to the CD45.2 allele. B) Engineered adenine base editors can convert A to G. To convert CD45.2 to CD45.1 two options were identified: Option 1: a sgRNA (gray bar) was designed to be used with Cas9 SaKKH (PAM highlighted by dotted black bar). This will convert the A at position 5 of the base editing window to a G. Option 2: a sgRNA (gray bar) to be used with Cas9 VQR. PAM highlighted by dashed black bar. This will convert the A at position 6 of the base editing window to G. Options 1 and 2 will convert the CD45.2 allele to the CD45.1 allele.

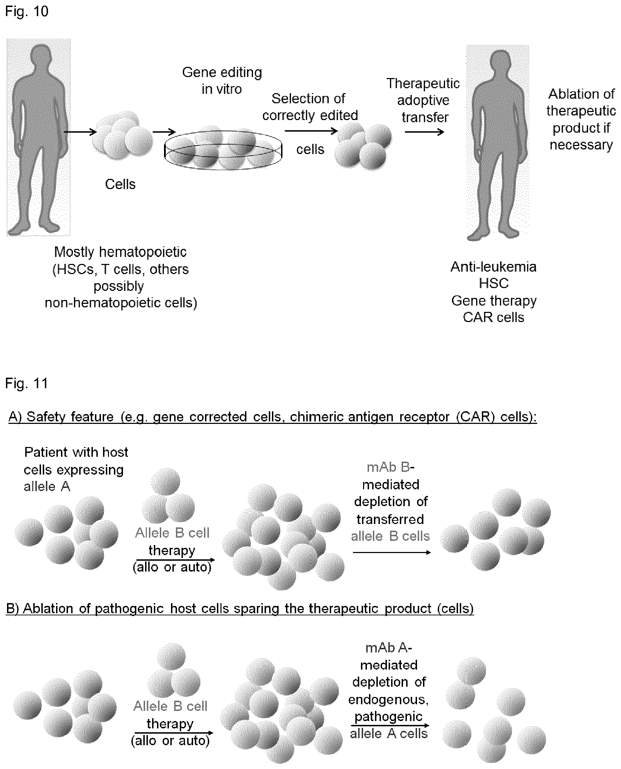

Fig. 10 shows the concept behind the optional ablation of the transferred cells. Cells are taken from a patient, then edited (ex vivo) to convert one allele to another one. Succssfully edited cells are selected to infuse a pure population of cells carrying the epitope necessary for selective depletion. Allele engineering has therefore added the option to ablate the transferred cells if needed.
Fig. 11 shows applications for selective depletion of transferred or host cells in vivo.
Fig. 12 shows a proof-of-concept experiment for selective ablation in vivo. Model for in vivo ablation: 1) Reconstitution of immunodeficient mice lacking all T cells (Rag KO) with a 1:1 mix of purified CD45.2+ and CD45.1+ T cells (congenic cells, not edited cells). 2) Antibody-mediated depletion (non-selective (anti-CD4) compared to selective (anti-CD45.2)). Anti-CD45.2 without toxin or coupled to toxin (Saporin). 3) Analysis 1 week later.
Fig. 13 shows selective depletion of CD45.2+ cells in vivo: Quantification of T cells in blood.

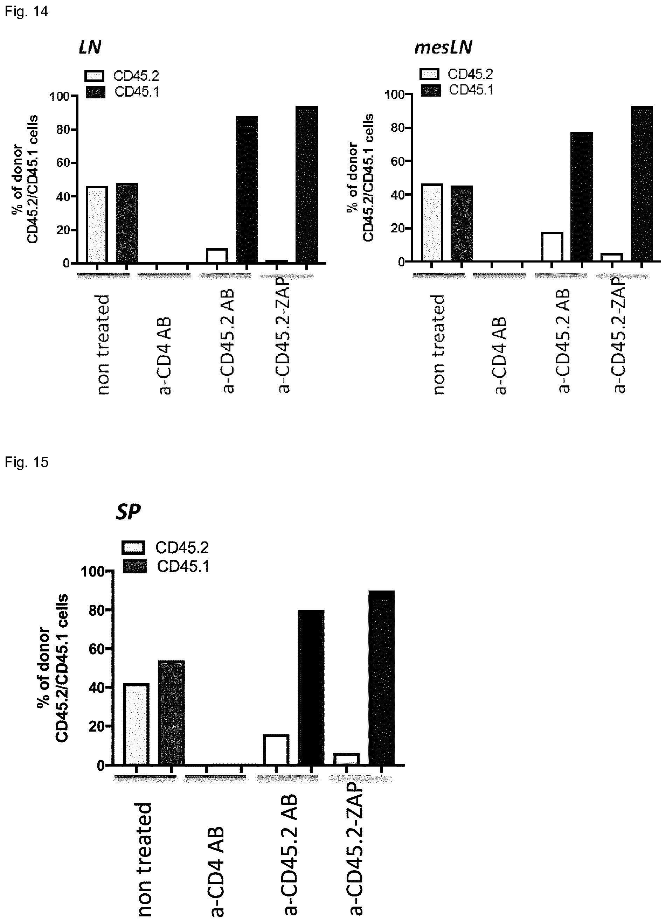

Fig. 14 shows selective depletion of CD45.2+ cells in vivo: Quantification of relative numbers of T cells in lymphoid organs. Same setup as in Fig. 12 but analysis of lymph nodes (LN) and mesenteric lymph nodes (mesLN).
Fig. 15 shows selective depletion of CD45.2+ cells in vivo: Quantification of relative numbers of T cells in lymphoid organs. Same setup as in Fig. 12 but analysis of spleen (SP).

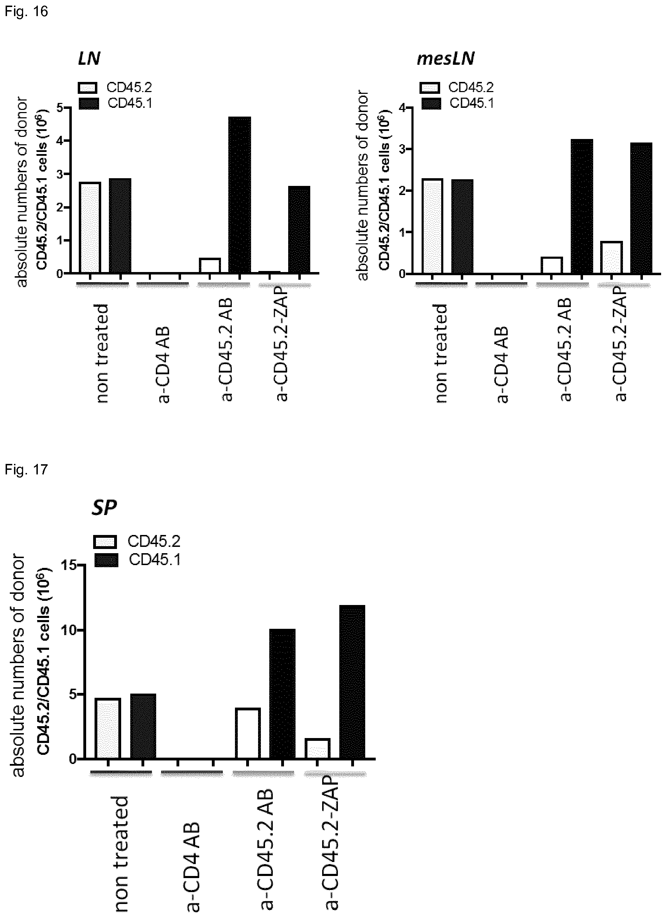

Fig. 16 shows selective depletion of CD45.2+ cells in vivo: Quantification of absolute numbers of T cells in lymphoid organs. Same setup as in Fig. 12 but analysis of lymph nodes (LN) and mesenteric lymph nodes (mesLN).
Fig. 17 shows selective depletion of CD45.2+ cells in vivo: Quantification of absolute numbers of T cells in lymphoid organs. Same setup as in Fig. 12 but analysis of spleen (SP).

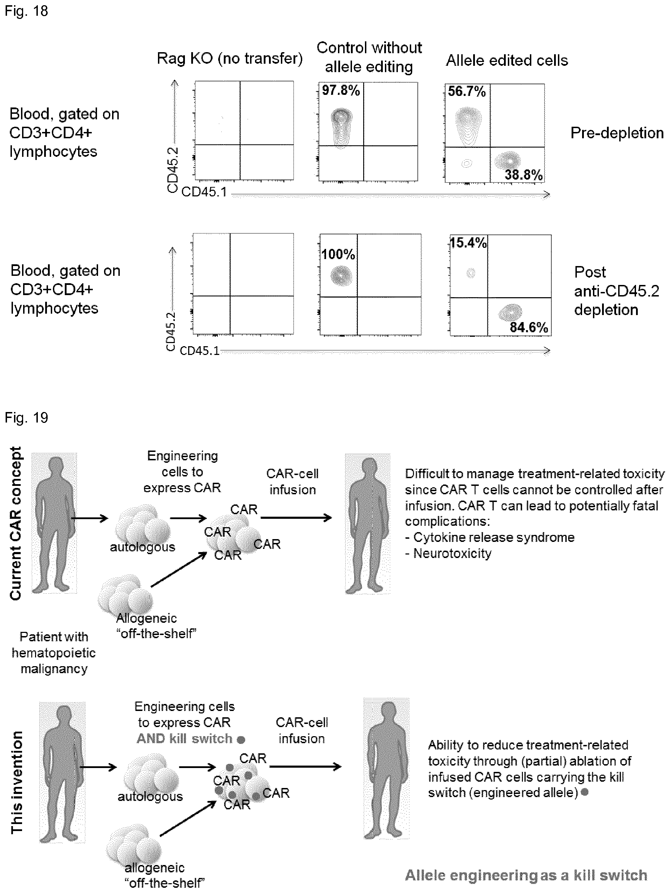

Fig. 18 shows a second proof-of-concept experiment for selective in vivo ablation of allele edited cells. Experimental setup: 1) Electroporation of CD45.2 T cells ex vivo to engineer to CD45.1 2) Adoptive transfer to T cell deficient mice (Rag KO); (no purity selection before transfer) 3) Weeks later quality control to demonstrate allele editing 4) Antibody-mediated depletion of CD45.2 cells leaving only edited cells. Conclusion: PoC demonstrating in vivo depletion of CD45.2+ host and unedited cells while sparing allele engineered CD45.1+ cells
Fig. 19 shows an application of the disclosure: Allele engineering increases safety, e.g. for CAR- T cell Therapy. Engineered cells express a first isoform of a cell surface protein (e.g. CD45) and a chimeric antigen receptor reactive to a target of interest, e.g. CD19 (CAR-19). While the CAR19 is engineered into the T cells the second CD45 allele is converted into the first CD45 allele to enable discrimination of the transferred CAR T cells from the non-engineered host cells. In the case of treatment related toxicity the pathogenic CAR-T cells can be selectively ablated to stop CAR-T related toxicity.
Fig. 20 shows the gene correction of scurfy cells and cells bearing the human Foxp3K276X mutation as well as enrichment of the relative frequency of gene-repaired cells when gating on an isoform-switched surrogate surface marker.
   A) Alignment of genomic DNA sequences of wildtype foxp3 (C57BL/6) (SEQ ID NO 028), the Foxp3 locus with a targeted mutation Foxp3K276X (SEQ ID NO 029) which introduces a premature stop codon and the Foxp3 locus of scurfy mice (B6.Cg-Foxp3sf/J) which harbor a spontaneous 2bp insertion leading to a frame-shift (SEQ ID NO 030). sgRNA binding sites (green line) and PAM sequences (black line). B) Protocol for gene editing of total CD4+ T cells from Foxp3K276X C57BL/6 mice. In vitro activation and electroporation (step 1) with plasmids encoding sgRNA targeting the Foxp3K276X mutation and a circular plasmid containing a 1kb wildtype (wt) Foxp3 repair template. Successfully transfected cells are isolated based on GFP expression (step 2). Cell expansion in vitro for gene editing in presence of rhIL-2, TGF-β alone or in combination with retinoic acid (RA) and cytokine neutralizing antibodies (anti-IL-4 and anti-IFNy for 7 days (step 3). C) Experimental setup as in B with total CD4+ T cells from control mice (WT) or Foxp3K276X mice. Flow cytometry of CD25 and Foxp3 expression (gated on live CD4+ T cells). Wildtype cells electroporated with empty px458 plasmid differentiate into CD4+Foxp3+CD25+ T cells (left panel), absence of Foxp3 differentiation in Foxp3K276X cells electroporated with sgRNA Foxp3K276X alone (middle panel) and restoration of Foxp3 protein expression in Foxp3K276X cells electroporated with sgRNA Foxp3K276X and 1kb Foxp3 dsDNA repair template (right panel). Top row: Foxp3 induction with TGF-β alone, bottom row: Foxp3 induction with TGF-β combined with RA. Compared to TGF-β alone the combination of TGF-β and RA leads to a higher frequency of Foxp3 expressing cells in those cells which have an intact Foxp3 locus (i.e. wildtype and repaired cells). Representative data from 2 experiments with Foxp3^{K276X} cells and one experiment with *Foxp3^{sf}*/J cells. D) Enrichment of gene-repaired Foxp3 expressing cells using multiplexed CD45 isoform switching as a surrogate marker. Experimental setup as in b but simultaneous electroporation of plasmids encoding 2 sgRNAs (sgRNA Foxp3K276X and sgRNACD45.2_R1) and two 1kb dsDNA templates (Foxp3 wildtype and CD45.1). Seven days later flow cytometry of CD45.2, CD45.1, CD25 and Foxp3 (gated on live CD4+ cells). Top panel: Pre-gating on CD45.1- cells (green line) and CD45.1+ cells (red line). Bottom panel: Enrichment of CD25+Foxp3+ cells in isoform switched CD45.1+ cells. Representative data from 2 experiments with Foxp3^{K276X} cells and one experiment with *Foxp3^{sf}*/J cells.

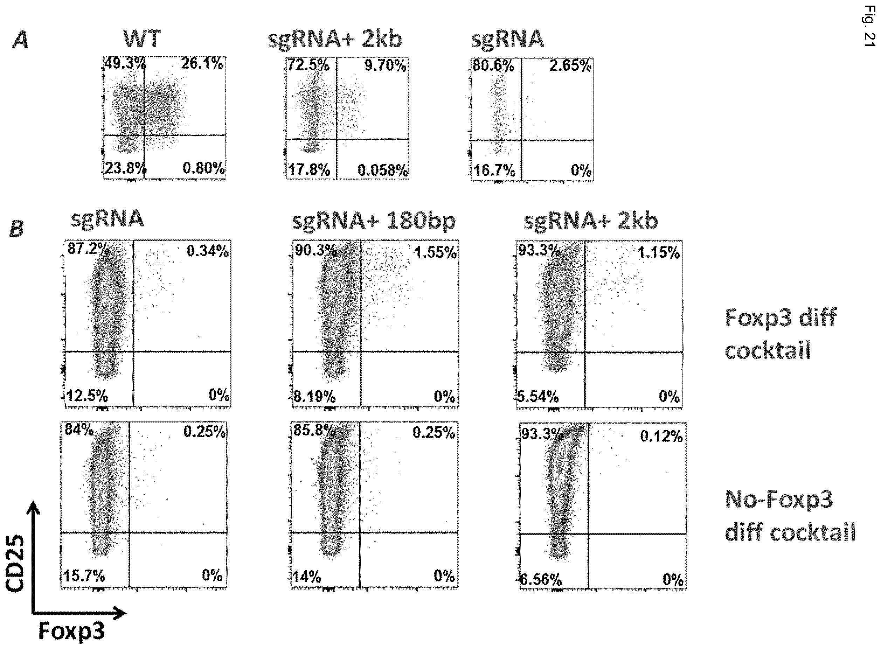

Fig. 21 shows repair of the Foxp3 gene using the plasmid based approach and the RNP based approach. A: CD4 T cells from Foxp3 KO mice were transfected with sgRNA plasmid alone or together with a Foxp3 wildtype HDR template. GFP+ and GFP- cells were sorted 24h post transfection (plasmid transfection) and immediately after cell sorting expanded until the end of the experiment in the presence of Foxp3 differentiation cocktail. B: CD4 T cells from Foxp3 KO mice were transfected with crRNA:tracrRNA/Cas9 RNP complex alone or +/- HDR templates (180bp ssDNA or 2kb plasmid). Total pool of RNPs transfected cells were expanded until the end of the experiment in the presence of Foxp3 differentiation cocktail.

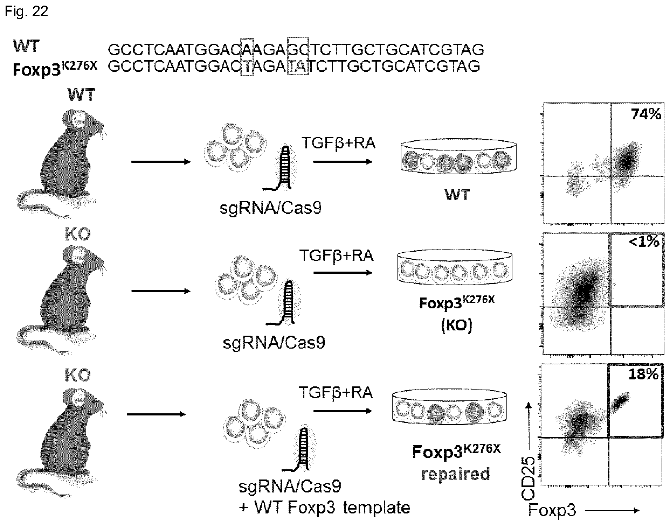

Fig. 22 shows successful Foxp3 gene repair in T cells in vitro. Upper row: Alignment of genomic DNA sequences of wt Foxp3 (C57BL/6) SEQ ID NO 031 and the Foxp3 locus with a targeted mutation Foxp3K276X SEQ ID NO 032 that introduces a premature stop codon. sgRNA binding sites (green line) and PAM sequences (black line). Total CD4+ T cells from wt control or Foxp3K276X mice were transfected. Flow cytometry of CD25 and Foxp3 expression (gated on live CD4+ T cells). Differentiation of wt cells electroporated with empty px458 plasmid into CD4+Foxp3+CD25+ T cells (upper panel), absence of Foxp3 differentiation in Foxp3K276X cells electroporated with sgRNAFoxp3K276X alone (middle panel) and restoration of Foxp3 protein expression in Foxp3K276X cells electroporated with sgRNAFoxp3K276X and 1kb Foxp3 dsDNA repair template (lower panel). Foxp3 induction with TGFbeta combined with RA.

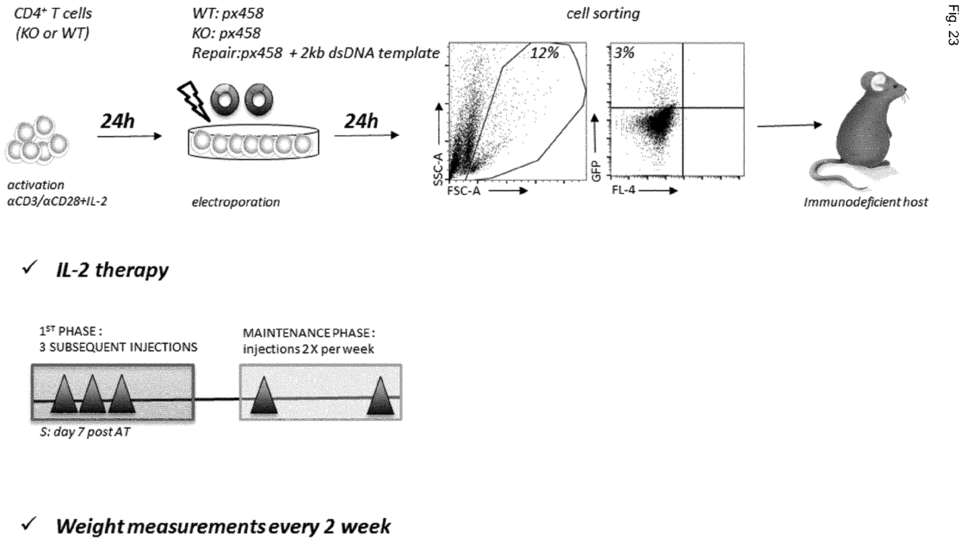

Fig. 23 shows a protocol for gene editing of total CD4+ T cells from Foxp3K276X C57BL/6 mice. In vitro activation and electroporation with plasmids encoding sgRNA targeting the Foxp3K276X mutation and a circular plasmid containing a 1kb wt Foxp3 repair template. Successfully transfected cells are isolated based on GFP expression. Cells are the transferred in vivo for Foxp3 differentiation followed by IL-2 regimen.
Fig. 24 shows the clinical phenotype of mice 14 weeks after AT of cells. WT and repair mice are disease free, transfer of KO cells results in skin inflammation and alopecia. Experimental setup as in Fig. 23.
Fig. 25 shows the clinical phenotype (T cell infiltration) in mice that received KO cells correlates with infiltration of CD4/CD3+ T cells in ear and tail skin. No or very low numbers of CD4/CD3+ T cells are found in ear and tail skin of mice that received WT or Repaired T cells. Data are depicted as % and absolute numbers.

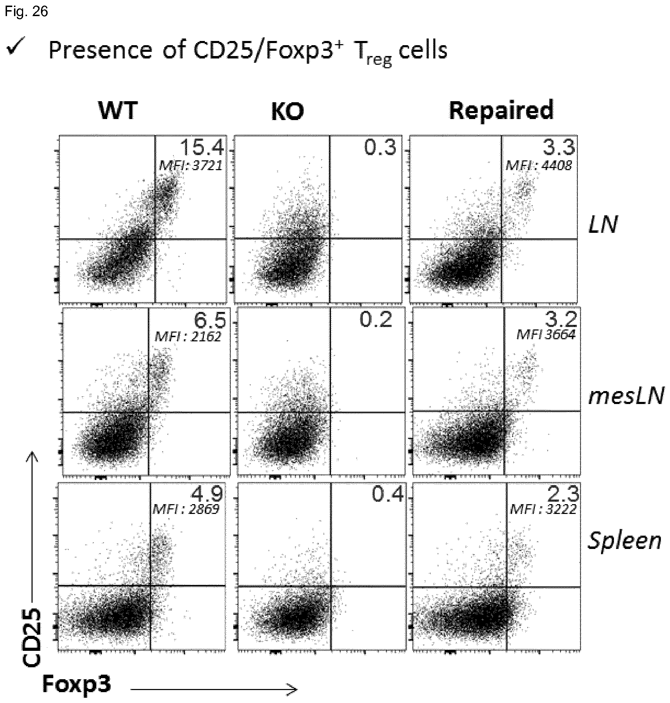

Fig. 26 shows the presence of CD25/Foxp3+ Treg cells in mice that received WT and repaired cells. No CD25/Foxp3+ T cells are found in mice that received KO cells. Data are displayed as % and MFI of Foxp3. Experimental setup as in Fig. 23.

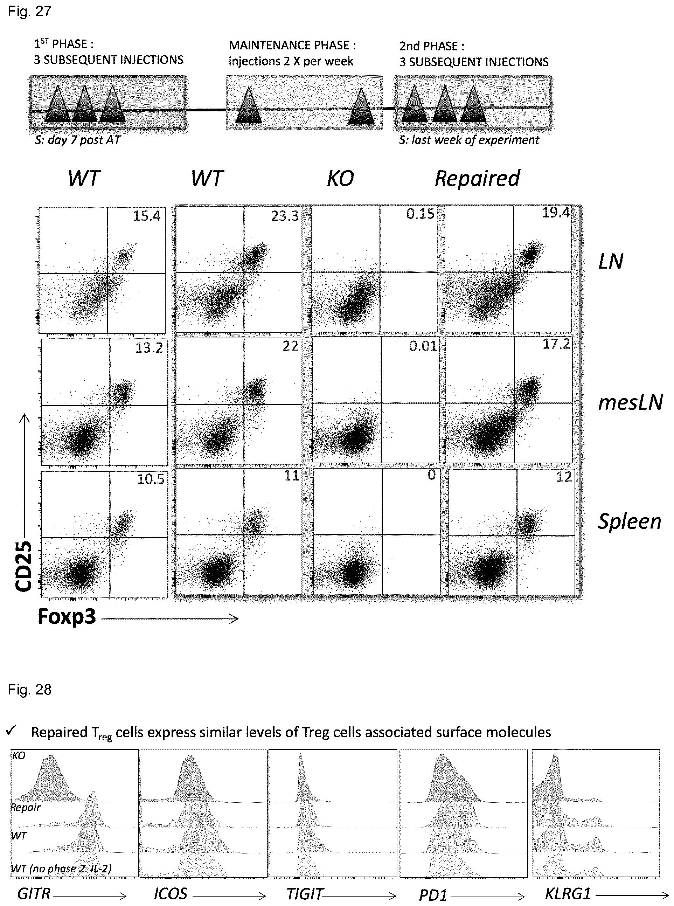

Fig. 27 shows that repaired Treg respond to IL-2. Additional IL-2 treatment during the last week of experiment results in increased presence of CD25/Foxp3+ Treg cells in mice that received WT and Repaired cells. No CD25/Foxp3+ T cells are found in mice that received KO cells. These data demonstrate that WT and Repaired Treg cells respond to IL-2 to similar extent.
Fig. 28 shows that WT and repaired CD25/Foxp3 Treg cells express similar levels of different cell surface markers (GITR, ICOS, TIGIT, PD1 and KLRG1). The combination of CD25 and additional markers (e.g. GITR) can be used to enrich for these populations. Foxp3-deficient T cells are phenotypically different, e.g. they lack GITR and KLRG1 expression.
Fig. 29 shows that AT of CD45.1+ WT cells rescues Foxp3 deficient mice and expands their life span up to 8 weeks (latest time point examined, no indications of disease). High proportion of WT Treg cells (50%) found in a Foxp3 KO mice suppress host T cells activation (lower % of CD44high cells). This demonstrates that WT T cells have the capacity to fully restore immune regulation and control scurfy disease. Furthermore, the Foxp3-deficient microenvironment boosts Foxp3-suficient T cells to expand massively.
Fig. 30 shows a CD4 depletion experiment in Foxp3 KO mice (lymphnode, LN). Combined gene repair in T cells with selective antibody depletion: Deplete pathogenic cells, then transfer gene-corrected T cells. CD4 depletion largely prevents scurfy disease and extends life expectancy significantly. The observation is supported by FACS data showing low or non-existent CD4 T cells (KO depleted compared to KO or WT mice without treatment).

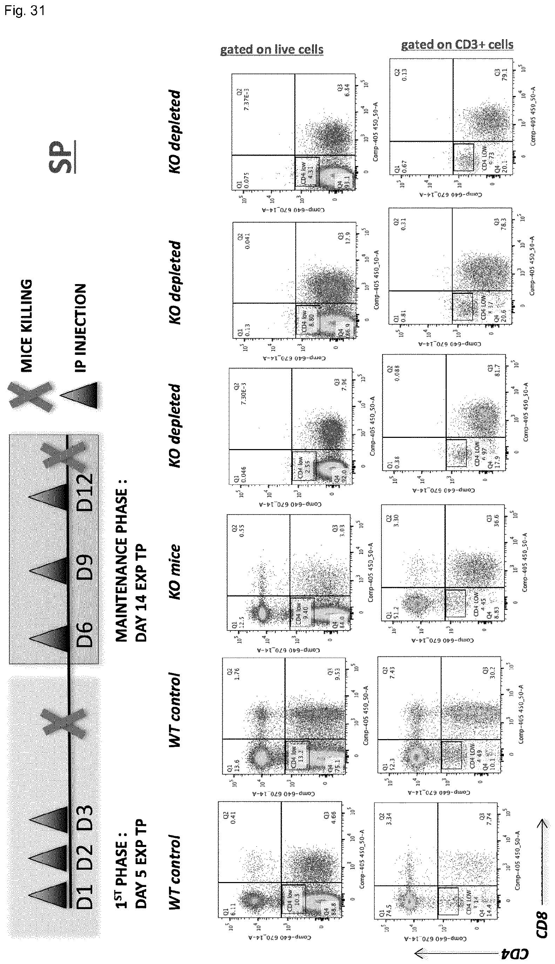

Fig. 31 shows a CD4 depletion experiment in Foxp3 KO mice (spleen, SP). Combined gene repair in T cells with selective antibody depletion: Deplete pathogenic cells, then transfer gene-corrected T cells. CD4 depletion largely prevents scurfy disease and extends life expectancy significantly. The observation is supported by FACS data showing low or non-existent CD4 T cells (KO depleted compared to KO or WT mice without treatment).

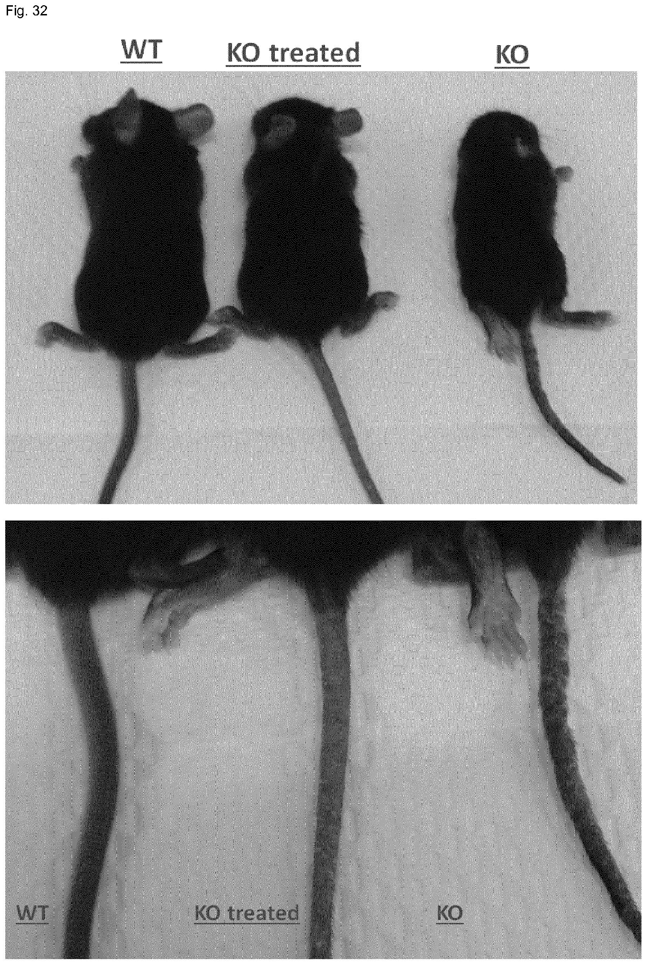

Fig. 32A shows that CD4 depletion prevents development of scurfy disease in Foxp3-deficient mice.
Fig. 32B shows that CD4 depletion largely prevents development of dermatitis in tail skin of Foxp3-deficient mice.

### Examples

### General considerations for the design of engineered mutations (allele engineering):

Engineering an allele of a gene by introducing a targeted small mutation, possibly single nucleotide or single amino acid mutation, with the purpose of changing the specific binding of a ligand and allowing the binding of a second, specific ligand can be useful as a general principle for therapeutic use. The mutation is designed in a way to preserve the function of the engineered protein as much as possible. The immunogenicity needs to be changed to be able to raise specific binding ligands such as monoclonal antibodies (mAb) or antibody-like molecules such as affimers, DARPINS, nanobodies etc. Such ligands need to be screened for specific binding and the nature of the immunogen needs careful design. At the same time, the mutation constitutes a minor antigenic change and therefore is unlikely to cause a strong immunologic reaction in vivo. Congenic markers in mice fulfill exactly these criteria. The difference between CD90.1 and CD90.2 as well as between CD45.1 and CD45.2 is a single nucleotide leading to a single amino acid difference. In both cases, this difference can be detected by specific mAbs resulting in a pair of mAbs for each gene. Although mAbs could be raised against those differences when congenic cells are transferred into matching congenic host mice, i.e. CD90.1+ cells into CD90.2+ host mice or vice versa and CD45.1+ cells into CD45.2+ hosts and vice versa the cells are not immunologically rejected. This means that this minor antigenic difference is tolerated by the immune system in vivo. Collectively, these properties have made congenically marked cells a very useful tool for immunologic research which has been used for decades. Immunologically these cells are used as surrogate cells for autologous transfers (because the cells are genetically identical except for this one mutation) but the congenic minor difference allows to discriminate transferred from host cells.

### Choice of a protein to be engineered:

To design a similar system for therapeutic use in humans several considerations need to be taken into account. The mutation can in principle be introduced into any gene encoding a protein. The expression pattern of the protein of interest will be relevant, i.e. ubiquitous or cell- or tissue-specific expression if desired. Proteins expressed on the surface can be most directly targeted and will therefore in most cases be the proteins of choice. Examples include but are not limited to the proteins characterized by the cluster of differentiation system CD1 to CD371) (Engel, J Immunol November 15, 2015, 195 (10) 4555-4563 and http://www.hcdm.org/). Other proteins of interest can be ubiquitously expressed proteins such as beta-2-microglobulin or constant parts of HLA-class I which are expressed on all cells, including non-immunologic cells. Alternatively, proteins expressed in specific cell types can be of interest, e.g. human CD45 for hematopoietic cells, human CD3 for T cells, constant regions of human T cell receptor components, constant regions of B cell immunoglobulins such as the kappa and lambda light chain or constant regions of the heavy chains, human CD4 or CD8 coreceptors or B cell markers such as CD19, CD20, CD21, CD22, CD23, costimulatory molecules such as CD28 or CD40, CD34 on hematopoietic stem cells or even specific isoforms expressed on subsets of cells such as CD45RA or CD45RO. In this latter case the mutation would be designed into the variable region (alternatively spliced exons) which differs between CD45RA and CD45RO. Engineering a mutation into a ubiquitously expressed molecule such as beta-2-microglobulin or HLA-I could serve as a unique system which can be used in virtually any mammalian cell, more specifically any human cell. This could be used to track and ablate the engineered cells while sparing the non-engineered host cells. Such a feature could e.g. be useful as a kill switch for cell therapy, including various types of stem cells (e.g. muscle stem cells), hepatic cells or cells derived from induced pluripotent stem cells (iPS). Engineering a mutation into a cell type-specific protein such as CD45 could be useful to target all cells from that specific tissue. In the case of the hematopoietic system, engineering human CD45 could serve to mark hematopoietic stem cells (HSC) which are used to replace the endogenous hematopoietic system. All progeny of those HSCs will harbor the same mutation engineered into the genome of the original HSCs. If the previous host hematopoietic system is removed by any means (e.g. irradiation, chemotherapy, depleting ligands such as mAb or antibody-like molecules, mAb coupled with toxins, cell-mediated ablation, e.g. CAR-T cell mediated ablation etc.) then the replacing hematopoietic system, even if autologous, could be discriminated by the engineered mutation.

Alternatively, it would allow the ablation of host cells while sparing the engineered cells.

### Design considerations:

- Expression pattern of the protein (ubiquitous versus cell- or tissue specific)
- Extracellular proteins: In most cases mutations will be engineered into the extracellular parts of proteins of interest which are accessible to specific ligands. The mutations can be engineered into constantly expressed exons or alternatively spliced exons if subset specific expression is desired.
- Conservation across species: conserved amino acids (aa) or structures are more likely to be functionally relevant and should therefore rather remain untouched. Mutations should rather be incorporated into less conserved regions.
- Chemical properties of the amino acids, e.g. polarity, electrical charge, hydrophobicity
- Similarity of amino acids: To preserve function the mutation should convert a given aa into a related aa but the changes still need to be detectable by specific ligands.
- Naturally occourring variations: mutations which correspond to amino acid variations observed at a give position in a multiple sequence alignememnts of members of a protein family (homologous sequences of different organisms) are likely to not affect the function and the structure of the protein, and can be slected to design mutations.
- Structural considerations: Structural data can assist in the rationale design of potential mutations. The amino acid to be engineered has to be accessible to ligand binding. Mutations in loops are functionally relatively well tolerated and are therefore of interest.
- Avoid known disease-causing mutations.
- Consider known human genetic variations: functionally tolerated single nucleotide polymorphisms (SNP)s are good candidate mutations.
- Sites important for secondary modifications such as glycosylations should not be mutated,
- Sites important for disulfide bonds should not be mutated.
- Sites important for reported interactions (e.g. hydrogen bonding, salt bridges, hydrophobic stacking interactions) should not be mutated, both protein internally but also protein-protein interactions for multiprotein complexes or receptor-ligand interactions.
- Consider previous successful systems such as murine CD90.1/CD90.2 (Q to R)
- Consider binding sites of known binding ligands such as known mAbs and computational modelling of complementarity determining regions (CDRs).
- Exclude known "active sites", e.g. catalytic sites of enzymes
- Avoid structurally very similar domains present in other human proteins, since they are likely to increase the risk for cross-specific ligands
- Consider immunogenicity: choose a site that is likely to result in a suitable peptide immunogen.

### Allele engineering as a generally applicable, platform-independent principle:

The inventors demonstrate and characterize how the CRISPR/Cas platform can be used to engineer a targeted point mutation for allele engineering followed by selective ablation. The inventors engineered two separate point mutations into two distinct genes and demonstrate that the engineered mutation can be used to track and/or selectively ablate engineered cells in vivo.

The inventors relied on the CRISPR/Cas9 system and a dsDNA template to achieve homology directed repair (HDR). However, the example used is just one way of introducing the mutation.

Alternatives include different types of nucleases such as zinc finger proteins, TALENs or other naturally occurring or engineered CRISPR/Cas systems such as Cas9, Cpf-1, high fidelity nucleases or nucleases with engineered PAM dependencies (Komor, Cell, 2017). Moreover, the described principle is independent of the form of delivery of the nucleases. They can be delivered as a plasmid, mRNA, recombinant protein, recombinant protein in complex with a guide RNA (i.e. ribonuclear complex, RNP), split recombinase or as an integrating or non-integrating virus, e.g. retrovirus, lentivirus, baculovirus or other viral delivery platform. The delivery modality can encompass electroporation or other forms such as lipofection, nanoparticle delivery, cell squeezing or physical piercing. In addition, the HDR template can be a ssDNA or dsDNA in the form of short ssDNA, long ssDNA or a circular or linear minicircle DNA, plasmid DNA or a viral DNA template, e.g. adeno-associated virus (AAV). Depending on the target cell, specific AAV serotypes are used to deliver cargo to the cells. For human T cells and human hematopoietic stem cells HDR templates are often provided as AAV6 but endonuclease RNP along with short ssDNA HDR templates can also successfully be used.

Thus, the mode how the mutation is introduced can be flexible.

### Allele engineering employing alternative approaches, e.g. base converters or base editors

The inventors demonstrated how a targeted dsDNA break followed by HDR can be exploited to introduce small, targeted, i.e. precise mutations for alleleic engineering. In addition, there are alternative approaches how designed point mutations could be engineered into the genome of a living cell. As an example, newly designed chimeric fusion proteins allow direct conversion of a target DNA base into another *(*Komor, Nature 2016*;* Nishida, Science 2016*;* Yang, Nat Comm, 2016*;* Ma, Nat Meth 2016*).* Enzymes such as deaminases can be fused to DNA binding modules such as (but not limited to) zinc finger proteins, TALENS or CRISPR/Cas systems. Naturally occurring cytidine deaminases (APOBEC1, APOBEC3F, APOBEC3G) and activation induced deaminase (AID) or the AID ortholog PmCDA1 (from sea lamprey) can convert cytidines (C) to uracils (U) in DNA. The DNA replication machinery will treat the U as T if DNA replication occurs before U repair. This leads to a conversion of a C:G to a T:A base pair (Yang, Nat Comm, 2016*;* Komor, Nature 2016*).* Therefore, several groups have developed engineered chimeric proteins with deaminases fused to DNA binding modules which are used to bring the deaminase to a specific genomic locus. For instance the CRISPR/Cas system can be used as a delivery system when an engineered, catalytically dead (dCas9) version of the Cas9 nuclease is used. This approach has successfully been used to target fused effector molecules to specific genomic loci. Applications include targeting fluorescent proteins to specific loci or bringing transcriptional transactivators or repressors to specific genomic loci to control specific gene expression (Wang, Ann Rev Biochem, 2016). Fusing a cytidine deaminase or AID to a nickase Cas9 and additional engineering to improve the base editing efficiency allows direct targeted base conversion (Komor, Nature 2016; Nishida, Science 2016)*.* Under certain circumstances this could have advantages over the HDR approach since base editing neither induces a dsDNA break nor requires the delivery of a DNA HDR template. Therefore this alternative approach could lead to fewer indels and thus might be a safe or even safer alternative to HDR based genome engineering. In addition, delivery of the base editor as mRNA or RNP could be sufficient and could be less toxic for certain cell types. For human T cells Cas9 RNPs offer a successful genome editing approach (Schumann, PNAS, 2015), therefore it can be anticipated that base converters in the form of RNPs might be particularly well-suited for hematopoietic cells including hematopoietic stem cells (HSCs) and T cells but in principle base conversion might be a suitable approach for allele engineering applicable to any cell, including mammalian cells. The introduced designed point mutation can then be used for downstream applications such as cell marking, cell tracking and selective ablation. Base editors delivered as RNPs successfully edited target nucleotides in mammalian cells, mouse and zebrafish embryos and the inner ear of live mice (Rees, Nat Comm, 2017*;* Kim, Nat Biotech, 2017 doi:10.1038/nbt.3816*).* Thus, these studies demonstrate the feasibility of specific, DNA-free base editing. However, the number of nucleotides in the (human) genome which are amenable to base conversion is more restricted than the HDR approach since it not only depends on the PAM sequence (for CRISPR/Cas-based base converters) but underlies additional restrictions. Although cytidine deaminase base editors can only convert C to T (or G to A), newly engineered adenine base editors can convert A to G (or T to C (Gaudelli, Nature 2017)). However, in addition to the PAM requirement, base conversion only occurs within a certain window, specified by the specific design and/or engineering of the fusion protein. Thus, compared to HDR-mediated introduction of a point mutation the number of editable nucleotides is much more restricted if base editors are used (Komor, Nature 2016). In addition, the window of base conversion encompasses several nucleotides. For instance, for the so-called base editor 3 (BE3) that uses S. pyogenes Cas9 (SpBE3, see Komor et al., Nature 2016) this window encompasses about 5 nucleotides. Thus, editing a single C to T within a stretch of multiple adjacent C nucleotides will not be possible and will lead to additional unwanted mutations which may change the amino acid of the resulting protein. Similar editing windows apply to adenine base editors. Furthermore, a different BE3 that is based on S. aureus Cas9, SaBE3, can result in detectable base editing at target Cs outside the canonical BE3 editing window. To address these limitations, newer base converter versions were engineered to have narrower editing windows and different PAM specificities than NGG (e.g. NGA, NGAG, NGCG, NNGRRT and NNNRRT) but the design of a specific base editor for a given single nucleotide conversion remains challenging (Kim, Nat Biotech 2017, doi:10.1038/nbt.3803). Although the new base converters expand the range of targetable nucleotides in the mammalian genome, not every nucleotide can be edited at will. Nevertheless, in order to take advantage of some of the benefits of base converters the inventors attempted to design a base editor for allele engineering of CD90.1 to CD90.2, CD90.2 to CD90.1, CD45.1 to CD45.2 or CD 45.2 to CD45.1. However, with the original NGG restricted base editors none of these conversions could be achieved with the available base editors. This is despite the fact that for both genes, CD90 and CD45, the allelic difference is encoded by a G to A substitution which in principle should be amenable to deaminase conversion. The only solution was to use a BE3 version based on SaCas9 containing 3 mutations that relax this Cas9 variant's PAM requirement to NNNRRT (Kleinstiver, Nature 2015*;* Kim, Nat Biotech, 2016 (doi:10.1038/nbt.3803*).* Choosing this base editor made it possible to design a base editor with a matching sgRNA that will convert the CD45.1 allele to the CD45.2 allele (Fig. 9A). However, this base editor will only work to convert CD45.1 to CD45.2 but not CD45.2 to CD45.1 conversion we designed hypothetical adenine base editors (adenine base editor fusions with Cas9 SaKKH or Cas9 VQR (Fig. 9B). In contrast, none of the CD90 allele conversions are possible, even with the currently existing base editor version.

### Methods

### Human T-cell Isolation and Antibodies

Human primary T cells were isolated from buffy coats (Blutspendezentrum, Basel) of healthy donors using Lymphoprep^{™} (Stemcell Technologies) density gradient. Naive CD4⁺T cells were pre-enriched with an Easysep Human naive CD4⁺ T-cell enrichment kit (Stemcell Technologies) according to the manufacturer's protocol. Alternatively, cord blood was used as source for PBMCs, without using naive T cells isolation step, given the high frequencies of naive T cells. Pre and post naive CD4⁺ T cells enrichment samples were stained with following antibodies in order to assess the purity: αCD4-FITC (OKT-4), αCD25-APC (BC96), αCD45RA-BV711 (HI100), αCD45RO-BV450 (UCHL1), αCD62L-BV605 (DREG-56), αCD3-PerCP (HIT3a) and Zombie-UV viability dye, all purchased at Biolegend.

In brief, for 1 buffy coat of 50ml: prepare 2 × 50 ml Falcon tubes with filter and add 16 ml of Lymphoprep to each tube, spin @ 300g for 1 min. Distribute the blood equally to both 50 ml filter tubes and top up with PBS to 50 ml. Spin @ 2000 rpm (acc 4, decc 1) for 15 min. Remove some of the serum and discard it. Carefully pool the white buffy coats to a fresh 50 ml Falcon tube. Add sterile PBS to the enriched PBMC fraction to approximately 50 ml and spin @ 300g for 5 min. Discard the supernatant and resuspend pellet with 10 ml PBS and top up to 50 ml and spin @ 300g for 5 min. Lyse the red blood cells, if needed, with red blood cell lysis buffer, before purification step.

### Human T-cell Transfection protocol

Naive CD4⁺ T cells or total PBMCs from blood or cord blood were used for transfection. For T cell activation, 2×10⁶ cells were plated in a 24-well plate (Corning) coated with monoclonal antibodies (mAbs) a-CD3 (hybridoma clone OKT3, 5 (*high*), 2.5(*medium*), 1 (*low*) µg/ml) and a-CD28 (hybridoma clone CD28. 2.5 (*high*), 1 (*medium*)*,* 0.5 (*low*) µg/ml, both from Biolegend) for 24h at 37°C with 5% CO₂ in the presence of 50IU/ml recombinant human Interleukin-2 (rhlL-2) (RD systems). 24h later T cells were harvested and washed with PBS. 2×10⁶ activated T cells were electroporated with the Amaxa Transfection System, T-020 program (for plasmid) or using Neon^{®} Transfection System (ThermoFisher) at the following conditions: voltage (1600V), width (10ms), pulses (3) 100µl tip, buffer R (for RNPs). Cells were transfected with 65µg of empty plasmid px458 (Addgene plasmid number: 48138) or crRNA:tracerRNA-Atto 550 (IDT) and Cas9 (Berkeley) complex. After electroporation cells were plated in 24-well plate in 650µl complete media with 50IU rhIL-2/ml in the presence of plate-bound mAbs at half the concentrations used for the initial activation, i.e. anti-CD3 (2.5, 1.25, 0.5 µg/ml) and anti-CD28 (1.25, 0.5, 0.25 µg/ml). The expression of GFP⁺ or Atto550⁺ cells were assessed 24h later by using Fortessa analyzer (BD Biosciences).

### CD45.2depletion experiment

CD4⁺ T cells were isolated from C57BL6 (CD45.2) mice and C57BL6 congenic (CD45.1) mice using EasySep Mouse CD4⁺ T Cell Isolation Kit (Stem cell Technologies). RAG KO mice were reconstituted with 1:1 ration of 10×10⁶ CD45.2 and CD45.1 donor CD4⁺ T cells. Same day as T cells transfer, mice also received intraperitoneal injections of PBS (non treated group) or a depleting a-CD4 Ab (clone GK1.5, 250µg) for 3 consecutive days. CD45.2-ZAP immunotoxins were prepared by combining CD45.2 biotinylated antibody (160 kDa MW, Biolegend) with streptavidin-SAP conjugate (2.8 saporin molecules per streptavidin, 135 kDa MW, Advanced Targeting Systems) in a 1:1 molar ratio and subsequently diluted in PBS immediately before use, same as described in the initial publication: (https://www.ncbi.nlm.nih.gov/pmc/articles/PMC5179034/). *In vivo* administration of immunotoxin or the control with non-conjugated CD45.2 antibody was performed by intravenous injections. One week later, blood, peripheral lymph nodes (LN), mesenteric LN (mesLN) and spleen (SP) were collected and cells were stained with the following fluorochrome-conjugated mAbs: anti-CD45.2 (104), anti-CD45.1 (A20), anti-CD4 (RM4-5), anti-CD3 (145-2C11) all from Biolegend. Samples were acquired on a BD Fortessa (BD Biosciences) and analyzed with FlowJo software (Tree Star).

## Claims

1. A human hematopoietic cell, expressing a first isoform of a surface protein, wherein said first isoform of said surface protein is functionally indistinguishable, but immunologically distinguishable from a second isoform of said surface protein, for use in a medical treatment of a human patient having cells expressing said second isoform of said surface protein, wherein said cell is administered prior to, concomitant with or after specific ablation of cells expressing said second isoform of said surface protein wherein said ablation of cells expressing said second isoform of said surface protein is performed by administration to said patient of an antibody, wherein said antibody is specifically reactive to said second isoform but not to said first isoform of said cell surface protein and wherein said treatment comprises changing a sequence encoding said surface protein gene in the patient's native genomic DNA by gene editing so as to obtain a first isoform having an insertion, deletion and/or substitution of 1, 2, 3, 4 or 5 amino acids in the amino acid sequence of compared to said second isoform of said surface protein.

2. The human cell for use in a medical treatment according to claim 1, wherein said surface protein is selected from CD1a, CD1b, CD1c, CD1d, CD1e, CD2, CD3, CD3d, CD3e, CD3g, CD4, CD5, CD6, CD7, CD8a, CD8b, CD9, CD10, CD11a, CD11b, CD11c, CD11d, CDwl2, CD13, CD14, CD15, CD15u, CD15s, CD15su, CD16, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD45, CD45RA, CD45RB, CD45RC, CD45RC, CD46, CD47, CD48, CD49a, CD49b, CD49c, CD49d, CD49e, CD49f, CD50, CD51, CD52, CD53, CD54, CD55, CD56, CD57, CD58, CD59, CD60a, CD60b, CD60c, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CD75, CD75s, CD77, CD79a, CD79b, CD80, CD81, CD82, CD83, CD84, CD85a, CD85d, CD85j, CD85k, CD86, CD87, CD88, CD89, CD90, CD91, CD92, CD93, CD94, CD95, CD96, CD97, CD98, CD99, CD99R, CD100, CD101, CD102, CD103, CD104, CD105, CD106, CD107a, CD107b, CD108, CD109, CD110, CD111, CD112, CD113, CD114, CD115, CD116, CD117, CD118, CD119, CD120a, CD120b, CD121a, CD121b, CD122, CD123, CD124, CD125, CD126, CD127, CD129, CD130, CD131, CD132, CD133, CD134, CD135, CD136, CD137, CD138, CD139, CD140a, CD140b, CD141, CD142, CD143, CD144, CDw145, CD146, CD147, CD148, CDw149, CD150, CD151, CD152, CD153, CD154, CD155, CD156a, CD156b, CD156c, CD157, CD158e, CD158i, CD158k, CD159a, CD159c, CD160, CD161, CD162, CD163, CD164, CD165, CD166, CD167a, CD167b, CD168, CD169, CD170, CD171, CD172a, CD172b, CD172g, CD173, CD174, CD175, CD175s, CD176, CD177, CD178, CD179a, CD179b, CD180, CD181, CD182, CD183, CD184, CD185, CD186, CD191, CD192, CD193, CD194, CD195, CD196, CD197, CDw198, CD199, CD200, CD201, CD202b, CD203c, CD204, CD205, CD206, CD207, CD208, CD209, CD210, CDw210b, CD212, CD213a1, CD213a2, CD215, CD217a, CD218a, CD218b, CD220, CD221, CD222, CD223, CD224, CD225, CD226, CD227, CD228, CD229, CD230, CD231, CD232, CD233, CD234, CD235a, CD235b, CD236, CD236R, CD238, CD239, CD240CE, CD240DCE, CD240D, CD241, CD242, CD243, CD244, CD245, CD246, CD247, CD248, CD249, CD252, CD253, CD254, CD256, CD266, CD267, CD268, CD269, CD270, CD271, CD272, CD273, CD274, CD275, CD276, CD277, CD278, CD279, CD280, CD281, CD282, CD283, CD284, CD286, CD289, CD290, CD292, CDw293, CD294, CD295, CD296, CD297, CD298, CD299, CD300a, CD300c, CD300e, CD301, CD302, CD303, CD304, CD305, CD306, CD307a, CD307b, CD307c, CD307d, CD307e, CD308, CD309, CD312, CD314, CD315, CD316, CD317, CD318, CD319, CD320, CD321, CD322, CD324, CD325, CD326, CD327, CD328, CD329, CD331, CD332, CD333, CD334, CD335, CD336, CD337, CD338, CD339, CD340, CD344, CD349, CD350, CD351, CD352, CD353, CD354, CD355, CD357, CD358, CD360, CD361, CD362, CD363, CD364, CD365, CD366, CD367, CD368, CD369, CD370, CD371, BCMA, an Immunoglobulin light chain (lambda or kappa), a HLA protein and β2-microglobulin,
in particular wherein said surface protein is selected from CD2, CD3, CD4, CD5, CD8, CD19, CD20, CD22, CD23, CD33, CD34, CD90, CD45, CD123, BCMA, an Immunoglobulin light chain (lambda or kappa), a HLA protein and β2-microglobulin.

3. The human cell for use in a medical treatment according to any one of the preceding claims, wherein said first isoform is not encoded in the patient's native genomic DNA.

4. The human cell for use in a medical treatment according to any one of the above claims, wherein said human cell is selected from the group consisting of a hematopoietic stem cell, a CD4+ T cell, a CD8+ T cell, a memory T cell, a regulatory T cell (T reg), a natural killer cell (NK), an innate lymphoid cell (ILC), a dendritic cell (DC), a B-lymphocyte, a mucosal-associated invariant T cell (MAIT) and a gamma delta T cell (γδ T), in particular a hematopoietic stem cell or a T cell.

5. The human cell for use in a medical treatment according to any one of the above claims, wherein said medical treatment comprises hematopoietic stem cell transplantation.

## Patentansprüche

1. Menschliche hämatopoetische Zelle, die eine erste Isoform von einem Oberflächenprotein exprimiert, wobei die erste Isoform des Oberflächenproteins funktionell ununterscheidbar, aber immunologisch unterscheidbar von einer zweiten Isoform des Oberflächenproteins ist, zur Verwendung in einer medizinischen Behandlung von einem menschlichen Patienten, der Zellen aufweist, die die zweite Isoform des Oberflächenproteins exprimieren, wobei die Zelle vor, gleichzeitig mit oder nach einer spezifischen Ablation von Zellen verabreicht wird, die die zweite Isoform des Oberflächenproteins exprimieren, wobei die Ablation von Zellen, die die zweite Isoform des Oberflächenproteins exprimieren, durch Verabreichung eines Antikörpers an den Patienten durchgeführt ist, wobei der Antikörper spezifisch bezüglich der zweiten Isoform, aber nicht bezüglich der ersten Isoform des Zelloberflächenproteins reaktiv ist, und wobei die Behandlung ein Verändern einer Sequenz, die das Oberflächenprotein-Gen in der nativen genomischen DNA des Patienten kodiert, durch Gen-Editing umfasst, um eine erste Isoform zu erhalten, die eine Insertion, Deletion und/oder Substitution von 1, 2, 3, 4 oder 5 Aminosäuren in der Aminosäuresequenz im Vergleich zu der zweiten Isoform des Oberflächenproteins aufweist.

2. Menschliche Zelle zur Verwendung in einer medizinischen Behandlung nach Anspruch 1, wobei das Oberflächenprotein ausgewählt ist aus CD1a, CD1b, CD1c, CD1d, CD1e, CD2, CD3, CD3d, CD3e, CD3g, CD4, CD5, CD6, CD7, CD8a, CD8b, CD9, CD10, CD11a, CD11b, CD11c, CD11d, CDw12, CD13, CD14, CD15, CD15u, CD15s, CD15su, CD16, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD45, CD45RA, CD45RB, CD45RC, CD45RO, CD46, CD47, CD48, CD49a, CD49b, CD49c, CD49d, CD49e, CD49f, CD50, CD51, CD52, CD53, CD54, CD55, CD56, CD57, CD58, CD59, CD60a, CD60b, CD60c, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CD75, CD75s, CD77, CD79a, CD79b, CD80, CD81, CD82, CD83, CD84, CD85a, CD85d, CD85j, CD85k, CD86, CD87, CD88, CD89, CD90, CD91, CD92, CD93, CD94, CD95, CD96, CD97, CD98, CD99, CD99R, CD100, CD101, CD102, CD103, CD104, CD105, CD106, CD107a, CD107b, CD108, CD109, CD110, CD111, CD112, CD113, CD114, CD115, CD116, CD117, CD118, CD119, CD120a, CD120b, CD121a, CD121b, CD122, CD123, CD124, CD125, CD126, CD127, CD129, CD130, CD131, CD132, CD133, CD134, CD135, CD136, CD137, CD138, CD139, CD140a, CD140b, CD141, CD142, CD143, CD144, CDw145, CD146, CD147, CD148, CDw149, CD150, CD151, CD152, CD153, CD154, CD155, CD156a, CD156b, CD156c, CD157, CD158e, CD158i, CD158k, CD159a, CD159c, CD160, CD161, CD162, CD163, CD164, CD165, CD166, CD167a, CD167b, CD168, CD169, CD170, CD171, CD172a, CD172b, CD172g, CD173, CD174, CD175, CD175s, CD176, CD177, CD178, CD179a, CD179b, CD180, CD181, CD182, CD183, CD184, CD185, CD186, CD191, CD192, CD193, CD194, CD195, CD196, CD197, CDw198, CD199, CD200, CD201, CD202b, CD203c, CD204, CD205, CD206, CD207, CD208, CD209, CD210, CDw210b, CD212, CD213a1, CD213a2, CD215, CD217a, CD218a, CD218b, CD220, CD221, CD222, CD223, CD224, CD225, CD226, CD227, CD228, CD229, CD230, CD231, CD232, CD233, CD234, CD235a, CD235b, CD236, CD236R, CD238, CD239, CD240CE, CD240DCE, CD240D, CD241, CD242, CD243, CD244, CD245, CD246, CD247, CD248, CD249, CD252, CD253, CD254, CD256, CD266, CD267, CD268, CD269, CD270, CD271, CD272, CD273, CD274, CD275, CD276, CD277, CD278, CD279, CD280, CD281, CD282, CD283, CD284, CD286, CD289, CD290, CD292, CDw293, CD294, CD295, CD296, CD297, CD298, CD299, CD300a, CD300c, CD300e, CD301, CD302, CD303, CD304, CD305, CD306, CD307a, CD307b, CD307c, CD307d, CD307e, CD308, CD309, CD312, CD314, CD315, CD316, CD317, CD318, CD319, CD320, CD321, CD322, CD324, CD325, CD326, CD327, CD328, CD329, CD331, CD332, CD333, CD334, CD335, CD336, CD337, CD338, CD339, CD340, CD344, CD349, CD350, CD351, CD352, CD353, CD354, CD355, CD357, CD358, CD360, CD361, CD362, CD363, CD364, CD365, CD366, CD367, CD368, CD369, CD370, CD371, BCMA, einer leichten Immunglobulinkette (Lambda oder Kappa), einem HLA-Protein und β2-Mikroglobulin, wobei das Oberflächenprotein insbesondere ausgewählt ist aus CD2, CD3, CD4, CD5, CD8, CD19, CD20, CD22, CD23, CD33, CD34, CD90, CD45, CD123, BCMA, einer leichten Immunglobulinkette (Lambda oder Kappa), einem HLA-Protein und β2-Mikroglobulin.

3. Menschliche Zelle zur Verwendung in einer medizinischen Behandlung nach einem der vorhergehenden Ansprüche, wobei die erste Isoform nicht in der nativen genomischen DNA des Patienten kodiert ist.

4. Menschliche Zelle zur Verwendung in einer medizinischen Behandlung nach einem der vorhergehenden Ansprüche, wobei die menschliche Zelle ausgewählt ist aus der Gruppe bestehend aus einer hämatopoetischen Stammzelle, einer CD4+-T-Zelle, einer CD8+-T-Zelle, einer Gedächtnis-T-Zelle, einer regulatorischen T-Zelle (T-reg), einer natürlichen Killerzelle (NK), einer angeborenen lymphatischen Zelle (ILC), einer dendritischen Zelle (DC), einem B-Lymphozyten, einer schleimhautassoziierten invarianten T-Zelle (MAIT) und einer Gamma-Delta-T-Zelle (γδ-T), insbesondere einer hämatopoetischen Stammzelle oder einer T-Zelle.

5. Menschliche Zelle zur Verwendung in einer medizinischen Behandlung nach einem der vorhergehenden Ansprüche, wobei die medizinische Behandlung eine hämatopoetische Stammzellentransplantation umfasst.

## Revendications

1. Cellule hématopoïétique humaine, exprimant une première isoforme d'une protéine de surface, dans laquelle ladite première isoforme de ladite protéine de surface est fonctionnellement indiscernable, mais immunologiquement discernable par rapport à une seconde isoforme de ladite protéine de surface, destinée à être utilisée dans un traitement médical d'un patient humain présentant des cellules exprimant ladite seconde isoforme de ladite protéine de surface, dans laquelle ladite cellule est administrée avant, en même temps que ou après une ablation spécifique de cellules exprimant ladite seconde isoforme de ladite protéine de surface dans laquelle ladite ablation de cellules exprimant ladite seconde isoforme de ladite protéine de surface est réalisée par l'administration audit patient d'un anticorps, dans laquelle ledit anticorps est spécifiquement réactif à ladite seconde isoforme mais pas à ladite première isoforme de ladite protéine de surface de cellule et dans laquelle ledit traitement comprend le changement d'une séquence codant ledit gène de protéine de surface dans l'ADN génomique natif du patient par une édition génomique de manière à obtenir une première isoforme ayant une insertion, une suppression et/ou une substitution de 1, 2, 3, 4 ou 5 acides aminés dans la séquence d'acides aminés par rapport à ladite seconde isoforme de ladite protéine de surface.

2. Cellule humaine destinée à être utilisée dans un traitement médical selon la revendication 1, dans laquelle ladite protéine de surface est sélectionnée parmi CD1a, CD1b, CD1c, CD1d, CD1e, CD2, CD3, CD3d, CD3e, CD3g, CD4, CD5, CD6, CD7, CD8a, CD8b, CD9, CD10, CD11a, CD11b, CD11c, CD11d, CDwl2, CD13, CD14, CD15, CD15u, CD15s, CD15su, CD16, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD45, CD45RA, CD45RB, CD45RC, CD45RC, CD46, CD47, CD48, CD49a, CD49b, CD49c, CD49d, CD49e, CD49f, CD50, CD51, CD52, CD53, CD54, CD55, CD56, CD57, CD58, CD59, CD60a, CD60b, CD60c, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CD75, CD75s, CD77, CD79a, CD79b, CD80, CD81, CD82, CD83, CD84, CD85a, CD85d, CD85j, CD85k, CD86, CD87, CD88, CD89, CD90, CD91, CD92, CD93, CD94, CD95, CD96, CD97, CD98, CD99, CD99R, CD100, CD101, CD102, CD103, CD104, CD105, CD106, CD107a, CD107b, CD108, CD109, CD110, CD111, CD112, CD113, CD114, CD115, CD116, CD117, CD118, CD119, CD120a, CD120b, CD212a, CD121b, CD122, CD123, CD124, CD125, CD126, CD127, CD129, CD130, CD131, CD132, CD133, CD134, CD135, CD136, CD137, CD138, CD139, CD140a, CD140b, CD141, CD142, CD143, CD144, CDw145, CD146, CD147, CD148, CDw149, CD150, CD151, CD152, CD153, CD154, CD155, CD156a, CD156b, CD156c, CD157, CD158e, CD158i, CD158k, CD159a, CD159c, CD160, CD161, CD162, CD163, CD 164, CD 165, CD 166, CD167a, CD167b, CD168, CD 169, CD 170, CD 171, CD172a, CD172b, CD172g, CD173, CD174, CD175, CD175s, CD176, CD177, CD178, CD179a, CD179b, CD180, CD181, CD182, CD183, CD184, CD185, CD186, CD191, CD192, CD193, CD194, CD195, CD196, CD197, CDw198, CD199, CD200, CD201, CD202b, CD203c, CD204, CD205, CD206, CD207, CD208, CD209, CD210, CDw210b, CD212, CD213a1, CD213a2, CD215, CD217a, CD218a, CD218b, CD220, CD221, CD222, CD223, CD224, CD225, CD226, CD227, CD228, CD229, CD230, CD231, CD232, CD233, CD234, CD235a, CD235b, CD236, CD236R, CD238, CD239, CD240CE, CD240DCE, CD240D, CD241, CD242, CD243, CD244, CD245, CD246, CD247, CD248, CD249, CD252, CD253, CD254, CD256, CD266, CD267, CD268, CD269, CD270, CD271, CD272, CD273, CD274, CD275, CD276, CD277, CD278, CD279, CD280, CD281, CD282, CD283, CD284, CD286, CD289, CD290, CD292, CDw293, CD294, CD295, CD296, CD297, CD298, CD299, CD300a, CD300c, CD300e, CD301, CD302, CD303, CD304, CD305, CD306, CD307a, CD307b, CD307c, CD307d, CD307e, CD308, CD309, CD312, CD314, CD315, CD316, CD317, CD318, CD319, CD320, CD321, CD322, CD324, CD325, CD326, CD327, CD328, CD329, CD331, CD332, CD333, CD334, CD335, CD336, CD337, CD338, CD339, CD340, CD344, CD349, CD350, CD351, CD352, CD353, CD354, CD355, CD357, CD358, CD360, CD361, CD362, CD363, CD364, CD365, CD366, CD367, CD368, CD369, CD370, CD371, BCMA, une chaîne légère d'immoglobuline (lambda ou kappa), une protéine HLA et une β2-microglobuline,
en particulier dans laquelle ladite protéine de surface est sélectionnée parmi CD2, CD3, CD4, CD5, CD8, CD19, CD20, CD22, CD23, CD33, CD34, CD90, CD45, CD123, BCMA, une chaîne légère d'immunoglobuline (lambda ou kappa), une protéine HLA et une β2-microglobuline.

3. Cellule humaine destinée à être utilisée dans un traitement médical selon l'une quelconque des revendications précédentes, dans laquelle ladite première isoforme n'est pas codée dans l'ADN génomique natif du patient.

4. Cellule humaine destinée à être utilisée dans un traitement médical selon l'une quelconque des revendications précédentes, dans laquelle ladite cellule humaine est sélectionnée parmi le groupe composé d'une cellule souche hématopoïétique, d'une cellule T CD4+, d'une cellule T CD8+, d'une cellule T à mémoire, d'une cellule T régulatrice (T reg), d'une cellule tueuse naturelle (NK), d'une cellule lymphoïde innée (ILC), d'une cellule dendritique (DC), d'un lymphocyte B, d'une cellule T invariante associée aux muqueuses (MAIT) et d'une cellule T gamma delta (γδ T), en particulier d'une cellule souche hématopoïétique ou d'une cellule T.

5. Cellule humaine destinée à être utilisée dans un traitement médical selon l'une quelconque des revendications ci-dessus, dans laquelle ledit traitement médical comprend une transplantation de cellule souche hématopoïétique.
